(19) Europäisches Patentamt / European Patent Office / Office européen des brevets

(11) **EP 4 671 292 A1**

(12) **EUROPEAN PATENT APPLICATION**
published in accordance with Art. 153(4) EPC

(43) Date of publication:
**31.12.2025 Bulletin 2026/01**

(21) Application number: **24760216.2**

(22) Date of filing: **14.02.2024**

(51) International Patent Classification (IPC):
*C08F 299/00* $^{(2006.01)}$    *A61K 9/06* $^{(2006.01)}$
*A61K 9/14* $^{(2006.01)}$    *A61K 38/39* $^{(2006.01)}$
*A61K 47/58* $^{(2017.01)}$    *A61K 47/69* $^{(2017.01)}$
*A61L 27/16* $^{(2006.01)}$    *A61P 43/00* $^{(2006.01)}$
*C08F 6/00* $^{(2006.01)}$    *C08J 7/00* $^{(2006.01)}$
*C08J 7/02* $^{(2006.01)}$    *C08K 5/05* $^{(2006.01)}$
*C08L 29/04* $^{(2006.01)}$    *C09D 129/04* $^{(2006.01)}$

(52) Cooperative Patent Classification (CPC):
**A61K 9/06; A61K 9/14; A61K 38/39; A61K 47/58;
A61K 47/69; A61L 27/16; A61P 43/00; C08F 6/00;
C08F 299/00; C08J 7/00; C08J 7/02; C08K 5/05;
C08L 29/04; C09D 129/04**

(86) International application number:
**PCT/JP2024/005065**

(87) International publication number:
**WO 2024/176915 (29.08.2024 Gazette 2024/35)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **22.02.2023 JP 2023026398**

(71) Applicant: **Kuraray Co., Ltd.**
**Kurashiki-shi, Okayama 710-0801 (JP)**

(72) Inventors:
• **HIRAI, Yusuke**
  **Tsukuba-shi, Ibaraki 305-0841 (JP)**
• **KOBAYASHI, Goro**
  **Tsukuba-shi, Ibaraki 305-0841 (JP)**
• **FUJITA, Akio**
  **Tsukuba-shi, Ibaraki 305-0841 (JP)**
• **AYANO, Satoru**
  **Tsukuba-shi, Ibaraki 305-0841 (JP)**

(74) Representative: **Müller-Boré & Partner
Patentanwälte PartG mbB
Friedenheimer Brücke 21
80639 München (DE)**

(54) **DRY HYDROGEL-FORMING ARTICLE, HYDROGEL, AND METHOD FOR PRODUCING DRY HYDROGEL-FORMING ARTICLE**

(57)    Disclosed is a dry hydrogel-forming article comprising a crosslinked product of a vinyl alcohol polymer and an alcohol solvent, wherein
a content of the alcohol solvent based on an amount of the dry hydrogel-forming article is 0.01 mass% or more and 15 mass% or less, and a content of water based on an amount of the dry hydrogel-forming article is 0 mass% or more and 70 mass% or less, and
the dry hydrogel-forming article has a sterility assurance level SAL of $1 \times 10^{-6}$ or less and a degree of cohesion of 10% or less.

EP 4 671 292 A1

## Description

TECHNICAL FIELD

The present invention relates to a dry hydrogel-forming article, a hydrogel, and a method for producing a dry hydrogel-forming article.

BACKGROUND ART

Polyvinyl alcohol (hereinafter, sometimes abbreviated as "PVA") is a water-soluble synthetic polymer having superior characteristics in hydrophilicity, reactivity, biodegradability, biocompatibility, low toxicity, and the like, and forms a hydrogel having high flexibility and strength by physical crosslinking or chemical crosslinking. Furthermore, a hydrogel conjugated with a biologically active substance (hereinafter, sometimes abbreviated as conjugated hydrogel) exhibits specific functions, and applications, such as an enzyme immobilization carrier (for example, Non-patent Document 1), an affinity carrier (for example, Non-patent Document 2), a cell culture substrate (for example, Non-patent Document 3), particles for vascular embolization (for example, Patent Document 1), and a cell culture carrier (for example, Patent Document 2), have been proposed.

Among these applications, particularly in applications for pharmaceutical products for use in the human body or in parts or the manufacture of medical equipment, the hydrogel must be used in a sterilized state. When an unsterilized hydrogel is used for the above applications, residual bacteria cause infection and fatally damage a living body or cells.

As a method for sterilizing a hydrogel, for example, the prior art cited above discloses that a conjugated hyrogel is irradiated with UV light for several hours or immersed in an aqueous ethanol solution for several hours and then used for cell culture (for example, Non-patent Documents 3 to 5). In addition, attempts have also been made to sterilize a hydrogel-forming composition before crosslinking (Patent Document 3).

PVA contained in such a hydrogel is crosslinked, and as a crosslinking method, for example, a method in which an enzyme is covalently bonded using carbonyl diimidazole to a hydrogel chemically crosslinked with glutaraldehyde (for example, Non-patent Document 1), a method in which a cell adhesion protein is covalently bonded to PVA with carboxy groups introduced (for example, Non-patent Documents 3 and 4), a method in which acrylamide groups are introduced to PVA to induce radical polymerization to crosslink the PVA, thereby producing a hydrogel with a cell adhesion peptide covalently bonded (for example, Non-patent Documents 6 and 7), and a method in which a cell adhesiveness gelatin is covalently bonded to PVA particles obtained by crosslinking PVA having a (meth)acryloyl group by radical polymerization (for example, Patent Document 2) are known.

PRIOR ART DOCUMENTS

PATENT DOCUMENTS

Patent Document 1: JP-A-2002-527206
Patent Document 2: WO 2020/105708 A
Patent Document 3: WO 2020/153382 A

NON-PATENT DOCUMENTS

Non-patent Document 1: Food Chemistry, 2001, Vol. 74, p. 281-288
Non-patent Document 2: Biotechnology Techniques, 1997, Vol. 11, p. 67-70
Non-patent Document 3: Journal of Biomedical Materials Research, 2001, Vol. 57, p. 217-223
Non-patent Document 4: Journal of Polymer Engineering, 2017, Vol. 37, p. 647-660
Non-patent Document 5: Journal of Applied Biomaterials, 1991, Vol. 2, p. 261-267
Non-patent Document 6: Biomaterials, 2002, Vol. 23, p. 4325-4332
Non-patent Document 7: Biomaterials, 2012, Vol. 33, p. 3880-3886

SUMMARY OF THE INVENTION

PROBLEMS TO BE SOLVED BY THE INVENTION

As a sterility assurance level (hereinafter, sometimes abbreviated as SAL) sufficient to be applied to the human body and the like, it is desirable to achieve SAL = $1 \times 10^{-6}$ or less. However, in a case where a hydrogel is sterilized by UV light irradiation or immersion in an aqueous ethanol solution as conventionally performed, sterilization up to SAL = $1 \times 10^{-6}$ or less is impossible although it is possible to attain sterilization to such an extent that the hydrogel can be used for a cell culture test in a short period of time. In a case where a sterilized hydrogel is applied to a cell culture process or a human body, there is a problem that the risk of infection by microorganisms is not eliminated.

In addition, in the case of performing such a treatment as autoclave sterilization at a high temperature or gamma ray sterilization in order to enhance the sterilization effect, it is known that in a hydrogel conjugated with a biologically active substance such as an enzyme, the biologically active substance or the like loses its original activity under a severe sterilization condition, and thus it is common not to perform such a treatment. Furthermore, it has also been found that when a hydrogel subjected to such a treatment as gamma ray sterilization is used for cell culture, cell adhesiveness may be deteriorated. In addition, it has been found that when a severe treatment such as gamma ray sterilization is performed in a state before crosslinking, crosslinking proceeds by the treatment, and it is difficult to obtain a hydrogel molded into a desired shape. In addition, it has also been found that the strength of the gel may be reduced as a result of by making crosslinked portions non-uniform.

While the present inventors have intensively studied a method for sterilizing a hydrogel, they have attempted to sterilize a hydrogel in a dried state. In this case, it has also been found that dried hydrogels may adhere to each other or may cohere.

The present invention has been devised in view of the above problems, and a challenge of the present invention is to provide a dry hydrogel-forming article which can provide a hydrogel sufficiently sterilized and is inhibited from cohesion during drying.

In a preferred embodiment, a challenge of the present invention is to provide a dry hydrogel-forming article which is sufficiently sterilized, can provide a hydrogel having sufficient strength, and is inhibited from cohesion during drying.

In another preferred embodiment, an object of the present invention is to provide a dry hydrogel-forming article which is sufficiently sterilized, can afford a hydrogel having high cell adhesiveness, and is inhibited from cohesion during drying.

SOLUTIONS TO THE PROBLEMS

As a result of intensive studies, the present inventors have found that the above challenges are overcome when sterilization is performed under the condition that the sterility assurance level SAL is $1 \times 10^{-6}$ or less in the state of a dry hydrogel-forming article dried such that the degree of cohesion is 10% or less with an alcohol solvent contained. Such a dry hydrogel-forming article can be produced, for example, by replacing water in a hydrogel with an alcohol solvent, then drying the hydrogel to remove the solvent, and performing sufficient sterilization in the state of a dry hydrogel-forming article.

That is, the present invention relates to the following [1] to [15].

[1] A dry hydrogel-forming article comprising a crosslinked product of a vinyl alcohol polymer and an alcohol solvent, wherein

a content of the alcohol solvent based on an amount of the dry hydrogel-forming article is 0.01 mass% or more and 15 mass% or less, and a content of water based on an amount of the dry hydrogel-forming article is 0 mass% or more and 70 mass% or less, and
the dry hydrogel-forming article has a sterility assurance level SAL of $1 \times 10^{-6}$ or less and a degree of cohesion of 10% or less.

[2] The dry hydrogel-forming article according to [1], wherein the following condition 1 is satisfied,
Condition 1:
when 100 parts by mass of water and 1 part by mass of the dry hydrogel-forming article are placed in a glass vial and stirred at a speed of 400 rpm for 3 hours using a stirrer chip having a length of 80% or more of a bottom diameter of the glass vial, a breakage rate of a swollen hydrogel-forming article is 5% or less.

[3] The dry hydrogel-forming article according to [1] or [2], wherein a degree of polymerization of the vinyl alcohol polymer is 450 or more.

[4] The dry hydrogel-forming article according to any one of [1] to [3], wherein the vinyl alcohol polymer has carboxy groups, and introduction rate of carboxy groups is 0.1 to 50 mol% based on all structural units constituting the vinyl alcohol polymer.

[5] The dry hydrogel-forming article according to any one of [1] to [4], wherein the vinyl alcohol polymer has ethylenically unsaturated groups, and an introduction rate of the ethylenically unsaturated groups is 0.01 to 10 mol% based on all structural units constituting the vinyl alcohol polymer.

[6] The dry hydrogel-forming article according to [5], wherein the ethylenically unsaturated groups are at least one selected from the group consisting of vinyl groups, (meth)acryloyl groups, (meth)acryloylamino groups, vinylphenyl groups, norbornenyl groups, and derivatives thereof.

[7] The dry hydrogel-forming article according to any of [1] to [6], wherein the dry hydrogel-forming article is an indefinitely shaped particle, a spherical particle, a finely molded article, an article with any shape formed with a 3D printer, a sheet, a fiber, a hollow fiber, a porous monolith, or a coated article.

[8] The dry hydrogel-forming article of any of [1] to [7], wherein the dry hydrogel-forming article is a spherical particle.

[9] The dry hydrogel-forming article according to any one of [1] to [8], wherein a biologically active substance is conjugated.

[10] The dry hydrogel-forming article according to any one of [1] to [9], wherein a biologically active substance is conjugated to a crosslinked product of a vinyl alcohol polymer by covalent bonding.

[11] A hydrogel being a swollen product of the dry hydrogel-forming article according to any one of [1] to [10].

[12] The hydrogel of [11], wherein the hydrogel is a spherical particle having a particle size of 10 to 5000 $\mu$m.

[13] A method for producing the dry hydrogel-forming article according to any one of [1] to [10], comprising the steps of:

preparing a preliminary hydrogel including a crosslinked product of a vinyl alcohol polymer;

immersing the preliminary hydrogel in an alcohol solvent, and then drying the preliminary hydrogel to obtain a dry preliminary hydrogel; and

sterilizing the dry preliminary hydrogel until a sterility assurance level SAL is $1 \times 10^{-6}$ or less.

[14] The method for producing the dry hydrogel-forming article according to [13], wherein radiation sterilization is performed in the step of sterilizing until the sterility assurance level SAL is $1 \times 10^{-6}$ or less.

[15] The method for producing the dry hydrogel-forming article according to [14], wherein a radiation dose in the radiation sterilization is 8.2 kGy or more.

EFFECTS OF THE INVENTION

According to the present invention, it is possible to provide a dry hydrogel-forming article which can provide a sufficiently sterilized hydrogel and is inhibited from cohesion during drying.

DETAILED DESCRIPTION

1. Dry hydrogel-forming article

<Dry hydrogel-forming article>

As a first embodiment, the present invention provides a dry hydrogel-forming article comprising a crosslinked product of a vinyl alcohol polymer and an alcohol solvent, wherein a content of the alcohol solvent based on an amount of the dry hydrogel-forming article is 0.01 mass% or more and 15 mass% or less, and a content of water based on an amount of the dry hydrogel-forming article is 0 mass% or more and 70 mass% or less, and the dry hydrogel-forming article has a sterility assurance level SAL of $1 \times 10^{-6}$ or less and a degree of cohesion of 10% or less.

<Vinyl alcohol polymer and method for producing the same>

As to the crosslinked product of a vinyl alcohol polymer contained in the dry hydrogel-forming article of the present invention, the vinyl alcohol polymer can be produced by saponifying polyvinyl ester obtained by polymerizing a vinyl ester monomer, thereby converting an ester group in the polyvinyl ester into a hydroxy group. Examples of the vinyl ester monomer include aliphatic vinyl esters, such as vinyl formate, vinyl acetate, vinyl propionate, vinyl n-butyrate, vinyl isobutyrate, vinyl pivalate, vinyl versatate, vinyl caproate, vinyl caprylate, vinyl caprate, vinyl laurate, vinyl myristate, vinyl palmitate, vinyl stearate, and vinyl oleate; and aromatic vinyl esters, such as vinyl benzoate. These monomers may be used singly, or two or more thereof may be used in combination. Among the vinyl ester monomers, aliphatic vinyl esters are preferable, and vinyl acetate is more preferable from the viewpoint of production cost. That is, the polyvinyl ester is preferably polyvinyl acetate obtained by polymerizing vinyl acetate.

The polyvinyl ester may contain a structural unit derived from a monomer other than the vinyl ester monomer as necessary as long as the effects of the present invention are not impaired. Examples of such other monomers include $\alpha$-

olefins, such as ethylene, propylene, n-butene, and isobutylene; acrylic acid or salts thereof; alkyl acrylates, such as methyl acrylate, ethyl acrylate, n-propyl acrylate, i-propyl acrylate, n-butyl acrylate, i-butyl acrylate, t-butyl acrylate, 2-ethylhexyl acrylate, dodecyl acrylate, and octadecyl acrylate; methacrylic acid or salts thereof; alkyl methacrylates, such as methyl methacrylate, ethyl methacrylate, n-propyl methacrylate, i-propyl methacrylate, n-butyl methacrylate, i-butyl methacrylate, t-butyl methacrylate, 2-ethylhexyl methacrylate, dodecyl methacrylate, and octadecyl methacrylate; acrylamide derivatives, such as acrylamide, N-methylacrylamide, N-ethylacrylamide, N,N-dimethylacrylamide, diacetoneacrylamide, acrylamidopropane sulfonic acid or salts thereof, acrylamidopropyldimethylamine or salts or quaternary salts thereof, and N-methylolacrylamide or derivatives thereof; methacrylamide derivatives, such as methacrylamide, N-methylmethacrylamide, N-ethylmethacrylamide, methacrylamidopropane sulfonic acid or salts thereof, methacrylamidopropyldimethylamine or salts or quaternary salts thereof, and N-methylolmethacrylamide or derivatives thereof; N-vinylamide derivatives, such as N-vinylformamide and N-vinylacetamide; vinyl ethers, such as methyl vinyl ether, ethyl vinyl ether, n-propyl vinyl ether, i-propyl vinyl ether, n-butyl vinyl ether, i-butyl vinyl ether, t-butyl vinyl ether, dodecyl vinyl ether, and stearyl vinyl ether; nitriles, such as acrylonitrile and methacrylonitrile; vinyl halides, such as vinyl chloride and vinyl fluoride; vinylidene halides, such as vinylidene chloride and vinylidene fluoride; allyl compounds, such as allyl acetate and allyl chloride; maleic acid or salts, esters or anhydrides thereof; vinyl silyl compounds, such as vinyltrimethoxysilane; and isopropenyl acetate. These monomers may be used singly, or two or more thereof may be used in combination.

The method for saponifying the polyvinyl ester is not particularly limited, but the saponification can be performed by the same method as conventionally used. For example, an alcoholysis method or a hydrolysis method using an alkali catalyst or an acid catalyst can be applied. Among them, a saponification reaction using methanol as a solvent and a caustic soda (NaOH) catalyst is simple and preferable.

The degree of polymerization of the vinyl alcohol polymer is preferably 450 or more, more preferably 500 or more, still more preferably 550 or more, and particularly preferably 600 or more from the viewpoint of suppressing embrittlement of the crosslinked product of the vinyl alcohol polymer during sterilization treatment and swelling with water. In addition, the degree of polymerization of the vinyl alcohol polymer is preferably 10,000 or less, more preferably 5,000 or less, and still more preferably 3,500 or less from the viewpoint of suppressing an increase in viscosity of the aqueous solution when a crosslinked product of the vinyl alcohol polymer is produced and improving processability. Two or more vinyl alcohol polymers differing in degree of polymerization may be mixed and used. The degree of polymerization of the vinyl alcohol polymer in the present description refers to a degree of polymerization measured in accordance with JIS K 6726:1994. Specifically, it can be determined from the limiting viscosity measured in water at 30°C after saponification and purification of the raw material PVA.

The degree of saponification of the vinyl alcohol polymer is preferably 50 mol% or more, more preferably 60 mol% or more, and still more preferably 65 mol% or more from the viewpoint of improving the solubility in water of the vinyl alcohol polymer. The upper limit of the degree of saponification may be 100 mol% or less, or 99 mol% or less. In the present description, the degree of saponification of the vinyl alcohol polymer means a proportion (mol%) of the number of moles of a vinyl alcohol unit accounting for in the total number of moles of a structural unit that can be converted into the vinyl alcohol unit by saponification (for example, a vinyl acetate unit) and the vinyl alcohol unit both in the raw material PVA, and can be measured in accordance with JIS K 6726:1994.

The 4 mass% viscosity at 20°C of the vinyl alcohol polymer is preferably 0.5 to 110 mPa·s, more preferably 1 to 80 mPa·s, and still more preferably 2 to 60 mPa·s. When the viscosity is within the above range, the ease of production of the hydrogel is improved, and the strength of the conjugated hydrogel can be improved. The viscosity in the present description refers to a viscosity of an aqueous solution containing 4 mass% of a vinyl alcohol polymer measured at a temperature of 20°C using a B-type viscometer (rotation speed: 12 rpm) in accordance with the rotational viscometer method of JIS K 6726:1994.

<Crosslinked product of vinyl alcohol polymer>

The dry hydrogel-forming article of the present invention includes a crosslinked product of the vinyl alcohol polymer. The crosslinked product may be one obtained by physically crosslinking a vinyl alcohol polymer, or may be one crosslinked by covalent bonding. The total amount of the structural units derived from vinyl alcohol and the structural units derived from a vinyl ester relative to all the structural units constituting the vinyl alcohol polymer used in the present invention is preferably 80 mol% or more, more preferably 90 mol% or more, and still more preferably 95 mol% or more.

Examples of the method for physically crosslinking a vinyl alcohol polymer include a method in which an aqueous solution of the vinyl alcohol polymer is frozen and then melted, and a method in which the vinyl alcohol polymer is dissolved and heated in a mixed solvent of dimethyl sulfoxide and water, and the heated solution is cooled to room temperature. Examples of the method for crosslinking a vinyl alcohol polymer by covalent bonding include a method using a polyfunctional crosslinking agent that reacts with side chain hydroxy groups of a vinyl alcohol polymer, and a method involving introducing functional groups into a vinyl alcohol polymer by copolymerization, post-modification, or the like and making them react. From the viewpoint of the stability of the hydrogel during swelling with water, it is preferable that the vinyl alcohol polymer is crosslinked by covalent bonding.

Examples of the method using a polyfunctional crosslinking agent that reacts with side chain hydroxy groups of a vinyl alcohol polymer include a method in which a polyfunctional aldehyde compound, such as glyoxal, malondialdehyde, or glutaraldehyde is reacted under acidic conditions (polyacetal crosslinking), a method in which a polyfunctional epoxy compound, such as epichlorohydrin or ethylene glycol diglycidyl ether, is reacted under alkaline conditions (polyether crosslinking), and a method in which a polyfunctional carboxylic acid compound, such as maleic acid or succinic acid, is reacted (polyester crosslinking).

The method involving introducing functional groups into a vinyl alcohol polymer by copolymerization and making them to react may be a method involving copolymerizing, in the process of producing the vinyl alcohol polymer, a vinyl ester monomer with a polymerizable monomer that is other than any vinyl ester monomer and that has a reactive substituent other than a hydroxy group, then saponifying the resulting copolymer to afford copolymerized, modified polyvinyl alcohol, which may hereinafter be referred to as "copolymerized, modified PVA", and then adding a multifunctional crosslinking agent capable of reacting with functional groups such as carboxy groups present in the copolymerized, modified PVA or amino groups present in the copolymerized, modified PVA. The copolymerized, modified PVA having carboxy groups may be referred to as "carboxylic acid-modified PVA", and the copolymerized, modified PVA having amino groups may be referred to as "amino-modified PVA".

Examples of the monomer to constitute the carboxylic acid-modified PVA include $\alpha,\beta$-unsaturated carboxylic acids, such as (meth)acrylic acid, maleic acid, fumaric acid, and itaconic acid; alkyl (meth)acrylates, such as methyl (meth) acrylate and ethyl (meth)acrylate; $\alpha,\beta$-unsaturated carboxylic acid anhydrides, such as maleic anhydride and itaconic anhydride; and derivatives thereof. A hydrogel can be obtained by copolymerizing a vinyl ester monomer with a monomer to constitute the above-described carboxylic acid-modified PVA, then saponifying the resulting copolymer to give carboxylic acid-modified PVA, and combining, for example, a multifunctional crosslinking agent capable of reacting with the introduced carboxy group, such as a multifunctional epoxy compound, such as epichlorohydrin or ethylene glycol diglycidyl ether, a multifunctional amino compound, such as ethylenediamine, polyethylene imines, or polyallylamine, and a carbodiimide condensing agent, such as 1-(3-dimethylaminopropyl)-3-ethylcarbodiimide hydrochloride.

The carboxy groups introduced into the carboxylic acid-modified PVA can be conjugated by covalently bonding a biologically active substance having an amino group with an amide linkage (-CONH-).

For the amino-modified PVA, a hydrogel can be obtained by copolymerizing, for example, a vinyl ester monomer and N-vinylformamide, then saponifying the resulting copolymer, and combining a multifunctional crosslinking agent capable of reacting with the introduced amino groups, such as a multifunctional epoxy compound as described above, a multifunctional carboxylic acid compound, such as succinic acid or maleic acid, and the aforementioned carbodiimide condensing agent.

An example of the method involving introducing functional groups into a vinyl alcohol polymer by post-modification and making them react may be a method of introducing ethylenically unsaturated groups into side chains of a vinyl alcohol polymer. The ethylenically unsaturated groups introduced can easily afford a hydrogel by inducing a polymerization reaction by adding an additive such as a radical initiator. The introduction of ethylenically unsaturated groups is preferably performed via side chains, terminal functional groups, or the like of the vinyl alcohol polymer, and it is more preferable to make a compound containing an ethylenically unsaturated group, which may hereinafter be abbreviated as "ethylenically unsaturated group-containing compound", to react with a hydroxy groups of side chains of the vinyl alcohol polymer.

Examples of the ethylenically unsaturated group-containing compound to be made to react with hydroxy groups which are side chains of the vinyl alcohol polymer include (meth)acrylic acid or a derivative thereof, such as (meth)acrylic acid, (meth)acrylic anhydride, (meth)acrylic acid halides, and (meth)acrylic acid esters, and (meth)acryloyl groups can be introduced by making these compounds to undergo an esterification reaction or a transesterification reaction in the presence of a base.

Examples of the ethylenically unsaturated group-containing compound to be made to react with hydroxy groups which are side chains of the vinyl alcohol polymer include compounds containing an ethylenically unsaturated group and a glycidyl group in the molecule, such as glycidyl (meth)acrylate and allyl glycidyl ether. By making these compounds undergo an etherification reaction in the presence of a base, (meth)acryloyl groups or allyl groups can be introduced into the vinyl alcohol polymer.

Furthermore, examples of the ethylenically unsaturated group-containing compound to be made to react with 1,3-diol groups of the vinyl alcohol polymer include compounds containing an ethylenically unsaturated group and an aldehyde group in the molecule, such as acryl aldehyde (acrolein), methacryl aldehyde (methacrolein), 5-norbornene-2-carboxaldehyde, 7-octenal, 3-vinylbenzaldehyde, and 4-vinylbenzaldehyde. By making these compounds to undergo an acetalization reaction in the presence of an acid catalyst, ethylenically unsaturated groups can be introduced into the raw material PVA. More specifically, for example, norbornenyl groups or vinylphenyl groups can be introduced into the raw material PVA by making 5-norbornene-2 carboxaldehyde, 3-vinylbenzaldehyde, 4-vinylbenzaldehyde, or the like to undergo an acetalization reaction. In addition, (meth)acryloylamino groups can be introduced into the vinyl alcohol polymer by making N-(2,2-dimethoxyethyl) (meth)acrylamide or the like to react. As a method for introducing ethylenically unsaturated groups into the vinyl alcohol polymer, a reaction other than the reactions recited as examples above can be used, and two or more reactions may be used in combination.

Examples of a method for introducing the ethylenically unsaturated groups further include a method in which reactive substituents such as carboxy groups present in carboxylic acid-modified PVA or amino groups present in amino-modified PVA are made to react with an ethylenically unsaturated group-containing compound. For the carboxy groups of the carboxylic acid-modified PVA, for example, methacryloyl groups can be introduced by generating ester linkages by making glycidyl methacrylate react under acidic conditions. For the amino groups of the amino-modified PVA, for example, acryloylamino groups can be introduced by making acrylic acid anhydride undergo an amidation reaction in the presence of a base and, for example, vinyloxycarbonyl groups can be introduced by making divinyl adipate undergo an amidation reaction. As a method for introducing ethylenically unsaturated groups via a copolymerized, modified PVA, a reaction other than the reactions recited as examples above can be used, and two or more reactions may be used in combination.

As the vinyl alcohol polymer having ethylenically unsaturated groups, a vinyl alcohol polymer in which ethylenically unsaturated groups are introduced via hydroxy groups of side chains of the raw material PVA such as 1,3-diol groups is preferable from the viewpoint of ease of production, and a vinyl alcohol polymer resulting from making (meth)acrylic acid or a derivative thereof undergo an esterification reaction or a transesterification reaction to hydroxy groups of side chains of the raw material PVA, or a vinyl alcohol polymer resulting from making a compound containing an ethylenically unsaturated group and an aldehyde group in the molecule undergo an acetalization reaction to 1,3-diol groups of a vinyl alcohol polymer is more preferable.

In one preferred embodiment of the present invention, the vinyl alcohol polymer in the crosslinked product of the vinyl alcohol polymer is preferably a vinyl alcohol polymer having carboxy groups from the viewpoint of appropriately maintaining the swellability of a hydrogel with a solvent (particularly water) and maintaining physical properties such as gel strength, and from the viewpoint of improving the conjugation density of a biologically active substance or the like. In this embodiment, the introduction rate of carboxy groups is preferably 50 mol% or less, more preferably 30 mol% or less, and still more preferably 15 mol% or less based on all the structural units constituting the vinyl alcohol polymer. From the viewpoint of maintaining the conjugation density of the biologically active substance high and exhibiting a high function, the introduction rate is preferably 0.1 mol% or more, more preferably 0.5 mol% or more, and still more preferably 1.0 mol% or more. In particular, it is expected that the hydrogel is distributed or stored in a dry state until it is used, but if once the hydrogel is dried, the hydrogel may be hard to return to the original swollen state. The vinyl alcohol polymer is preferably a vinyl alcohol polymer having carboxy groups at the aforementioned introduction rate of carboxy groups because even when a dry hydrogel-forming article once dried is swollen to afford a hydrogel, the degree of swelling returns to the original degree and a high-strength hydrogel can be produced.

In another preferred embodiment of the present invention, the vinyl alcohol polymer in the crosslinked product of the vinyl alcohol polymer is preferably a vinyl alcohol polymer having ethylenically unsaturated groups from the viewpoint of suppressing embrittlement of a hydrogel and promoting a crosslinking reaction. In this embodiment, the ethylenically unsaturated groups is more preferably at least one selected from the group consisting of vinyl groups, (meth)acryloyl groups, (meth)acryloylamino groups, vinylphenyl groups, norbornenyl groups, and derivatives thereof.

In the above embodiment, the introduction rate of ethylenically unsaturated groups is preferably 10 mol% or less, more preferably 5 mol% or less, and still more preferably 3 mol% or less based on all the structural units constituting the vinyl alcohol polymer from the viewpoint of suppressing embrittlement of a hydrogel. Meanwhile, from the viewpoint of promoting the crosslinking reaction to rapidly form a hydrogel and improving the elastic modulus of the resulting hydrogel, the introduction rate is preferably 0.01 mol% or more, more preferably 0.1 mol% or more, and still more preferably 0.5 mol% or more.

In a more preferred embodiment of the present invention, the vinyl alcohol polymer in the crosslinked product of the vinyl alcohol polymer is more preferably a vinyl alcohol polymer having carboxy groups and ethylenically unsaturated groups from the same viewpoint as described above. In this case, the introduction rate of carboxy groups and the introduction rate of ethylenically unsaturated groups are more preferably the introduction rates described above, respectively.

From the viewpoint of improving the mechanical strength of a hydrogel and introducing functional groups different from the hydroxy groups of PVA, the vinyl alcohol polymer may further contain a monomer. Examples of the monomer include acrylamides, such as acrylamide, N-isopropylacrylamide, 2-acrylamido-2-methylpropane sulfonic acid, and N,N-dimethylacrylamide; $\alpha,\beta$-unsaturated carboxylic acids, such as (meth)acrylic acid, crotonic acid, itaconic acid, maleic acid, and fumaric acid; water-soluble radical polymerizable monomers, such as vinylpyridine, hydroxyethyl (meth)acrylate, styrene sulfonic acid, and polyethylene glycol mono(meth)acrylate; and crosslinking agents having two or more ethylenically unsaturated groups in the molecule, such as N,N'-methylenebisacrylamide, ethylene glycol di(meth)acrylate, diethylene glycol di(meth)acrylate, and polyethylene glycol di(meth)acrylate. From the viewpoint of improving the mechanical strength of a hydrogel, the content of the monomer is preferably 50 mass% or less, more preferably 30 mass% or less, and still more preferably 10 mass% or less based on the vinyl alcohol polymer having ethylenically unsaturated groups.

When the vinyl alcohol polymer has ethylenically unsaturated groups, the vinyl alcohol polymer can be gelled by crosslinking the ethylenically unsaturated groups introduced into the vinyl alcohol polymer by active energy rays or heat, and thereby a crosslinked product of the vinyl alcohol polymer can be obtained. Examples of the active energy rays include gamma rays, ultraviolet rays, visible rays, infrared rays (heat rays), radio waves, alpha rays, beta rays, electron beams, plasma stream, ionizing radiation, and particle beams. When the vinyl alcohol polymer is crosslinked by ultraviolet rays, visible rays, infrared rays (heat rays) or the like among the active energy rays or by heat, it is preferable that the uncrosslinked polymer solution described later contains a radical polymerization initiator. Examples of the radical polymerization initiator include a photoradical polymerization initiator and a thermal radical polymerization initiator.

The photoradical polymerization initiator is not particularly limited as long as radical polymerization is initiated by irradiation with active energy rays, such as ultraviolet rays or visible light. However, from the viewpoint of washing and removing the photoradical polymerization initiator after crosslinking, those that exhibit solubility in water are preferable. Examples thereof specifically include 1-[4-(2-hydroxyethoxy)-phenyl]-2-hydroxy-2-methyl-1-propan-1-one (trade name: Omnirad2959, manufactured by IGM RESINS B.V.), $\alpha$-ketoglutaric acid, lithium phenyl(2,4,6-trimethylbenzoyl)phosphinate (trade name "L0290", manufactured by Tokyo Chemical Industry Co., Ltd.), and eosin Y.

The thermal radical polymerization initiator is not particularly limited as long as it initiates radical polymerization by heat, and examples thereof include azo initiators and peroxide initiators which are commonly used in radical polymerization. From the viewpoint of improving the transparency and physical properties of the vinyl alcohol polymer, a peroxide initiator that does not generate gas is preferable. From the viewpoint of washing and removing the thermal radical polymerization initiator after crosslinking as described above, those that exhibit solubility in water are preferable. Examples thereof specifically include inorganic peroxides, such as ammonium persulfate, potassium persulfate, and sodium persulfate.

Alternatively, a redox polymerization initiator combined with a reducing agent may be used. In the case of a redox polymerization initiator, crosslinking can be performed by stimulation such as mixing of a peroxided initiator and a reducing agent. As the reducing agent to be combined as the redox polymerization initiator, publicly-known reducing agents can be used, and among these, N,N,N',N'-tetramethylethylenediamine, sodium sulfite, sodium bisulfite, and sodium hydrosulfite, which are high in solubility in water, are preferable.

Also as the azo initiators, those that exhibit solubility in water are preferable, and examples thereof specifically include 2,2'-azobis[2-(2-imidazolin-2-yl)propane]dihydrochloride (trade name: VA-044), 2,2'-azobis[2-(2-imidazolin-2-yl)propane]disulfate dihydrate (trade name: VA-044B), 2,2'-azobis[2-methylpropionamidine]dihydrochloride (trade name: V-50), 2,2'-azobis[N-(2-carboxyethyl)-2-methylpropionamidine]tetrahydrate (trade name: VA-057), 2,2'-azobis[2-(2-imidazolin-2-yl)propane] (trade name: VA-061), 2,2'-azobis[2-methyl-N-(2-hydroxyethyl)propionamide] (trade name: VA-086), and 4,4'-azobis(4-cyanopentanoic acid) (trade name: V-501) (all produced by Wako Pure Chemical Corporation).

In a preferred embodiment of the present invention in which the vinyl alcohol polymer has ethylenically unsaturated groups, when a vinyl alcohol polymer in which the ethylenically unsaturated groups are vinyl groups is used, for example, the vinyl alcohol polymer may be crosslinked utilizing a thiol-ene reaction by adding a polythiol having two or more thiol groups in the molecule from the viewpoint of promoting crosslinking. As the polythiol, those that exhibit solubility in water are preferable, and examples thereof include polythiols having a hydroxy group, such as dithiothreitol; and polythiols containing an ether linkage such as terminal thiol products, such as 3,6-dioxa-1,8-octanedithiol, polyethylene glycol dithiol, and multi-arm polyethylene glycol.

As described above, an optimum method of crosslinking the vinyl alcohol polymer by covalent bonding can be selected, as necessary.

For example, methods using glutaraldehyde as a crosslinking agent are also conventionally known, but glutaraldehyde is highly toxic, and may adversely affect when slightly eluted from a chemically crosslinked hydrogel. Therefore, although a method using glutaraldehyde can be used in research applications, it is difficult to put such a method to practical use in pharmaceutical applications and the like. Therefore, from the viewpoint of improving safety, the crosslinked product of the dry hydrogel-forming article of the present invention is preferably not crosslinked with glutaraldehyde, and those crosslinked with glutaraldehyde are excluded.

From the viewpoint of the toxicity of the unreacted crosslinking agent or the condensing agent contained in the hydrogel and the ease of molding, a method involving introducing functional groups into the vinyl alcohol polymer by post-modification and making them react, particularly a method involving introducing ethylenically unsaturated groups into side chains of the vinyl alcohol polymer is preferable.

<Alcohol solvent>

The dry hydrogel-forming article of the present invention further includes an alcohol solvent. In the dry hydrogel-forming article of the present invention, the content of the alcohol solvent based on the amount of the dry hydrogel-forming article is 0.01 mass% or more and 15 mass% or less. When a dry hydrogel-forming article drying is produced by performing drying under the condition that the content of the alcohol solvent will fall within the above range, cohesion of the dry hydrogel-forming article can be prevented. The content of the alcohol solvent in the dry hydrogel-forming article is not particularly limited as long as it is within the above range, but is preferably 0.05 to 15 mass%, more preferably 0.07 to 15 mass%, and still more preferably 0.1 to 15 mass% from the viewpoint of easily decreasing the degree of cohesion.

The alcohol solvent is not particularly limited as long as it is a water-soluble alcohol, and examples thereof include monoalcohols such as methanol, ethanol, propanol, and isopropanol, and polyhydric alcohols such as ethylene glycol, diethylene glycol, triethylene glycol, and glycerin. As the alcohol solvent, only one solvent may be contained, or two or more solvents may be contained.

<Water>

In the present invention, the dry hydrogel-forming article is a hydrogel in a dry state, and may or may not contain water, but is an article that can be swollen by absorbing additional water to form a hydrogel. In the dry hydrogel-forming article of the present invention, the content of water based on the amount of the dry hydrogel-forming article is 0 mass% or more and 70 mass% or less. The content of water in the dry hydrogel-forming article is not particularly limited as long as it is within the above range, but is preferably 60 mass% or less, more preferably 50 mass% or less, still more preferably 45 mass% or less, and further preferably 40 mass% or less from the viewpoint that the lower the water content, the lower the capacity and the higher the transport efficiency.

A method for measuring the content of the alcohol solvent in the dry hydrogel-forming article may be a method of measuring the content from a weight change after completely volatilizing water-soluble alcohol can be mentioned because, for example, in a case where water contained in a hydrogel is replaced with an alcohol solvent and then dried, the solvent contained in the hydrogel can be regarded as the alcohol solvent. Specifically, the weight of particles dried at 37°C is measured, then the particles are further dried at 120°C, the weight after drying until the weight no longer changes is measured, and the content of the alcohol solvent can be calculated from the weight difference before and after the drying at 120°C by the following calculation formula (I).

$$\text{Alcohol solvent content} = ((\text{weight after drying at 37°C}) - (\text{weight after drying at 120°C}))/(\text{weight after drying at 37°C}) \qquad (I)$$

The content of the alcohol solvent in the dry hydrogel-forming article can also be measured by, for example, $^1$H-NMR together with the content of water. Details of the measurement conditions are as described in Examples.

In one embodiment of the present invention, the total content of water and the alcohol solvent in the dry hydrogel-forming article may be, for example, 0.01 mass% or more and 85 mass% or less, 0.05 mass% or more and 75 mass% or less, 0.07 mass% or more and 65 mass% or less, or 0.1 mass% or more and 55 mass% or less. In addition, the ratio of the content of the alcohol solvent to the total content of water and the alcohol solvent in the dry hydrogel-forming article may be preferably 50 mass% or more (or more than 50 mass%), more preferably 60 mass% or more, still more preferably 70 mass% or more, further preferably 80 mass% or more, and particularly preferably 90 mass% or more from the viewpoint of easily preventing cohesion. The upper limit of the ratio may be 100 mass% or less. The dry hydrogel-forming article of the present invention includes an alcohol solvent, and may or may not include water.

<Degree of cohesion>

The dry hydrogel-forming article of the present invention has a degree of cohesion of 10% or less. When the degree of cohesion exceeds 10%, the fixed and/or cohered dry hydrogel-forming article may remain cohered even at the time of swelling and may not uniformly swell, or the surface structure of the hydrogel may be broken when being separated at the time of collision or swelling. In addition, there also is a possibility that the cohered hydrogel is broken at the time of separation, and fragments of the hydrogel are generated. When such fragments are mixed at the time of use, for example, when the hydrogel is used for cell culture, the fragments may be taken into cells during culture or may be difficult to be separated from the cultured cells.

The degree of cohesion is determined by taking a certain weighed amount of dry hydrogel-forming articles, separating, by visual observation, cohered materials in which two or more of the dry hydrogel-forming articles are cohered, measuring the weight of the cohered materials separated, and calculating the degree of cohesion from the following formula (II).

$$\text{Degree of cohesion [\%]} = (\text{weight of cohered material/total weight}) \times 100 \cdots$$

(II)

The calculation of the degree of cohesion is performed using a measurement sample including a plurality of dry hydrogel-forming articles. Alternatively, for example, in the case of the dry hydrogel-forming article being a sheet, the degree of cohesion may be measured, using a dry hydrogel-forming article obtained by stacking and drying a plurality of sheets and drying, from the number of sheets adhering to each other.

A method for adjusting the contents of water and the alcohol solvent and the degree of cohesion within the above ranges may be a method in which a hydrogel containing water is produced, then the hydrogel is immersed in an alcohol solvent to replace water contained in the hydrogel with the alcohol solvent, and then the resulting hydrogel is dried. In addition, to adjust the sterility assurance level SAL to be described later to $1 \times 10^{-6}$ or less, it is conceivable to subject a hydrogel dried as described above to sterilization treatment by radiation sterilization (for example, gamma ray sterilization) or the like.

<Sterility assurance level SAL>

The dry hydrogel-forming article of the present invention has a sterility assurance level SAL of $1 \times 10^{-6}$ or less. If the sterility assurance level SAL exceeds $1 \times 10^{-6}$, a hydrogel that can be used for pharmaceutical applications or the like cannot be provided. When the dry hydrogel-forming article of the present invention satisfies the sterility assurance level SAL described above in the state of a dry hydrogel that can be used as a hydrogel when being swollen, high sterility can be obtained even at the time of final use. By adjusting the sterility assurance level SAL to $1 \times 10^{-6}$ or less by, for example, irradiation with gamma rays or the like in the state of a dry hydrogel-forming article, it is considered, though the reason is not clear, that generation of radicals can be suppressed and cell adhesiveness can be easily enhanced.

The sterility assurance level SAL can preferably be ensured by performing sterilization under the conditions defined by the $VD_{max}$ method. Details of the condition setting by the $VD_{max}$ method are as described in Examples, etc. The dry hydrogel-forming article of the present invention is an article satisfying the sterility assurance level described above, preferably an article sterilized by radiation, more preferably a gamma-ray-sterilized article, and still more preferably a gamma-ray-sterilized article with a sterilizing dose prescribed by the $VD_{max}$ method. Incidentally, a dry hydrogel-forming article including a crosslinked product obtained by crosslinking a vinyl alcohol polymer subjected to radiation sterilization (particularly gamma ray sterilization) before the crosslinking cannot be said to have sufficient moldability as a hydrogel, and may have insufficient strength, and thus is preferably excluded from the scope of the present invention.

<Breakage rate>

The dry hydrogel-forming article of the present invention preferably satisfies the following condition 1.

Condition 1:

when 100 parts by mass of water and 1 part by mass of the dry hydrogel-forming article are placed in a glass vial and stirred at a speed of 400 rpm for 3 hours using a stirrer chip having a length of 80% or more of a bottom diameter of the glass vial, a breakage rate of a swollen hydrogel-forming article is 5% or less.

When a dry hydrogel-forming article satisfies Condition 1, in other words, has a breakage rate of 5% or less as measured by the method described above, the strength of the dry hydrogel-forming article is high. Therefore, even when the dry hydrogel-forming article is subjected to sterilization treatment such that the sterility assurance level SAL will be $1 \times 10^{-6}$ or less, it is possible to maintain the strength of the resulting hydrogel, and it is considered that such a dry hydrogel-forming article is likely to maintain the degree of swelling when swollen again after drying. The breakage rate is preferably 4% or less, more preferably 3% or less, still more preferably 2% or less, and further preferably 1% or less from the viewpoint of improving the strength of the gel. The breakage rate is determined by observing the state of a solid component after stirring under the above conditions with a microscope, observing the solid component in a state where the solid component can be seen in 60% or more of the visual field, visually discriminating between the damaged portion and the non-damaged portion, and calculating the breakage rate from the ratio of the area of the damaged portion to the area of the entire solid component.

<Degree of swelling>

The degree of swelling of the dry hydrogel-forming article of the present invention is calculated from the weight $W_1$ g of the dry gel-forming article in a dry state and the weight $W_2$ g of the dry gel-forming substance in a state swollen by immersing 30 mg of the dry gel-forming substance in 5 mL of PBS (phosphate buffered saline) overnight using the following formula (III):

$$\text{Degree of swelling} = W_2/W_1 \text{ (III)},$$

and is preferably 5 or more, more preferably 6 or more, and still more preferably 8 or more. The upper limit of the degree of swelling is not particularly limited, but is preferably 40 or less, more preferably 30 or less, and still more preferably 20 or less from the viewpoint of the strength of the hydrogel.

<Shape of dry hydrogel-forming article>

The shape of the dry hydrogel-forming article is not particularly limited, but is preferably an indefinitely shaped particle, a spherical particle, a finely molded article, an article with any shape formed with a 3D printer, a sheet, a fiber, a hollow fiber, a porous monolith, or a coated article. From the viewpoint of suppressing cohesion, the dry hydrogel-forming article is preferably a spherical particle, and more preferably a spherical particle having a particle size of 10 to 5000 $\mu$m when swollen. The particle size of the dry hydrogel-forming article being a spherical particle may be 1 to 5000 $\mu$m. The dry hydrogel-forming article may be one dry hydrogel-forming article having the shape as described above, or may be a group of a plurality of dry hydrogel-forming articles (for example, a particle group), but is usually a group of a plurality of dry hydrogel-forming articles, and is preferably a group of 10 or more, more preferably 30 or more, still more preferably 50 or more, and further preferably 70 or more dry hydrogel-forming articles.

The aforementioned finely molded article is a molded article with fine unevenness or the like provided on the surface or inside thereof, and the size of fine processing is 10 to 1000 $\mu$m. The any shape molded with a 3D printer is, for example, any shape that can be molded with a 3D printer of a stereolithographic, ink jet, or nozzle extrusion 3D printer. The coated article refers to an item in which a conjugated hydrogel is provided on a substrate having such a shape as a sheet, a tray, a fiber, a hollow fiber, a porous monolith, a finely molded article, or an article with any shape molded with a 3D printer, and as a material of the substrate, such a material as glass, polyolefin, polymethyl methacrylate, polystyrene, polyester, polyolefin, a polyethylene vinyl alcohol copolymer, polyamide, or polyimide can be freely chosen. The dry hydrogel-forming article is preferably a dry hydrogel-forming particle from the viewpoint of preventing cohesion.

<Conjugation of biologically active substance>

In the dry hydrogel-forming article of the present invention, a biologically active substance may be conjugated with the crosslinked product of the vinyl alcohol polymer. When the biologically active substance is conjugated, cell adhesiveness can be enhanced when a hydrogel obtained by swelling the dry hydrogel-forming article is used for such applications as cell culture. In the case of the dry hydrogel-forming article of the present invention in which a biologically active substance is conjugated, it is possible to provide a hydrogel having high cell adhesiveness and being sufficiently sterilized.

Examples of the biologically active substance include cell adhesion proteins or peptides, such as gelatin, collagen, laminin, fibronectin, retronectin, vitronectin, elastin, and synthetic RGD peptide; growth factors, such as a fibroblast growth factor (FGF), an epithelial growth factor (EGF), and a vascular endothelial growth factor (VEGF); acidic polysaccharides containing glycosaminoglycan, such as heparin, hyaluronic acid, chondroitin sulfate, dermatan sulfate, or heparan sulfate; antibodies, hormones, serum proteins, antibody-binding proteins, such as albumin, macroglobulin, globulin, and protein A, pharmaceuticals, and enzymes, such as proteases, lipase, amylase, and cellulase. The biologically active substance is preferably one having a functional group capable of binding to the dry hydrogel-forming article. Examples of such a functional group include a primary or secondary amino group, a carboxy group, and a hydroxy group; a primary amino group or a carboxy group is preferable, and a primary amino group is more preferable. When a biologically active substance is conjugated with the dry hydrogel-forming article of the present invention, the biologically active substance may be one substance or may be a combination of two or more substances.

When the dry hydrogel-forming article of the present invention includes a crosslinked product of a vinyl alcohol polymer having carboxyl groups, the biologically active substance is preferably a biologically active substance having a primary or secondary amino group. The biologically active substance may be, for example, at least one selected from the group consisting of a cell adhesion protein, a cell adhesion peptide, a growth factor, an acidic polysaccharide, an antibody, a hormone, a serum protein, an antibody binding protein, a medicine, and an enzyme. Examples of the biologically active substance having an amino group (preferably a primary or secondary amino group) include cell adhesion proteins or peptides, such as gelatin, collagen, laminin, fibronectin, and synthetic RGD peptide; growth factors, such as a fibroblast growth factor (FGF), an epithelial growth factor (EGF), and a vascular endothelial growth factor (VEGF); antibody-binding proteins, such as antibodies, serum proteins, albumin, macroglobulin, globulin, and protein A; glycosaminoglycans, such as heparin, hyaluronic acid, chondroitin sulfate, dermatan sulfate, and heparan sulfate; medical products; and enzymes, such as protease, lipase, amylase, and cellulase. These biologically active substances are preferable because these biologically active substances contain an amino group, and when the dry hydrogel-forming article of the present invention includes a crosslinked product of a vinyl alcohol polymer having carboxyl groups, these biologically active substances are easily conjugated to the crosslinked product by amide linkages. As the biologically active substance, a biologically active polypeptide containing two or more amino acids is also preferable. The amino group may be either an aromatic amino group or an aliphatic amino group, but is preferably an aliphatic amino group, and is preferably a side chain amino group or an N-terminal amino group of lysine (Lys) of the biologically active polypeptide. The amino group contained in the biologically active substance may be either a primary amino group or a secondary amino group, and may include one or both of them. Both the primary amino group and the secondary amino group can form an amide linkage with a carboxy group that can be possessed by a crosslinked product of a vinyl alcohol polymer.

<Conjugate>

In one embodiment of the present invention, the dry hydrogel-forming article of the present invention is a conjugate with a biologically active substance. In the present embodiment, the dry hydrogel-forming article of the present invention may be a dry hydrogel-forming article merely containing the biologically active substance, or may be a conjugate in which a crosslinked product of a vinyl alcohol polymer included in the dry hydrogel-forming article and a biologically active substance are bound by covalent bonding, but is preferably a dry hydrogel-forming article in which a biologically active substance is covalently bonded. When the crosslinked product of the vinyl alcohol polymer and the biologically active substance form a covalent bond, the biologically active substance can be retained in the hydrogel to stably function.

As a method of bonding the hydrogel particle and the biologically active substance by covalent bonding, for example, it is possible to choose a method involving forming a covalent bond by activating a hydroxy group of PVA to react with a functional group of a biologically active substance, a method involving forming a covalent bond by making a carboxylic acid-modified PVA or an amino-modified PVA react with a functional group of a biologically active substance. From the viewpoint of the efficiency of a reaction, it is preferable to use an amino group, a carboxylic acid, or a thiol group is as the functional group of the biologically active substance, and a carboxylic acid is more preferable.

Examples of a specific method for activating a hydroxy group of PVA to introduce a biologically active substance include a method involving using a hydroxy group activating reagent, such as 1,1'-carbonyldiimidazole, di(N-succimidyl) carbonate, p-toluenesulfonyl chloride, 2,2,2-trifluoroethanesulfonyl chloride, and cyanuric chloride. When the hydroxy group activating reagent is made to react with a hydroxy group of PVA, a covalent bond can be formed with a functional group such as a primary or secondary amino group, a carboxy group, or a hydroxy group of a biologically active substance. It is also possible to use an acid anhydride reagent, such as succinic anhydride, which can introduce a carboxylic acid by ester-linking to the hydroxy group of the vinyl alcohol polymer, and an acetal reagent, such as 2,2-dimethoxyethylamine, which can introduce an amino group to a 1,3-diol group of the vinyl alcohol polymer by an acetal linkage. Such a carboxylic acid and an amino group can form an amide linkage using, for example, an amino group of a biologically active substance, a carboxylic acid, and the aforementioned carbodiimide condensing agent. For example, when an acid anhydride reagent such as succinic anhydride is made to react with a crosslinked vinyl alcohol polymer, a carboxy group (COOH) derived from succinic acid is introduced at a terminal, and this carboxy group and an amino group of a biologically active substance are condensed using a condensing agent such as 1,1'-carbonyldiimidazole, dicyclohexylcarbodiimide, or water-soluble carbodiimide, whereby the biologically active substance can be conjugated with the crosslinked vinyl alcohol polymer via an amide linkage.

A specific method for introducing a biologically active substance into a carboxylic acid-modified PVA or an amino-modified PVA may be a method involving forming an amide linkage using an amino group of the biologically active substance, a carboxylic acid, the aforementioned carbodiimide condensing agent, and the like as described above. Although the method given above as an example can be used, an optimum method among methods including other methods can be used depending on the application.

The conjugate may be formed after a step of crosslinking an uncrosslinked polymer solution as described below, or may be conjugated at the stage of the uncrosslinked polymer solution. The conjugation of the biologically active substance is preferably performed after the crosslinking step in order to avoid a decrease in activity or loss of activity of the biologically active substance due to crosslinking.

2. Method for producing dry hydrogel-forming article

The method for producing the dry hydrogel-forming article of the present invention is not particularly limited, and examples thereof include a production method including a step of preparing a preliminary hydrogel including a crosslinked product of a vinyl alcohol polymer (a preliminary hydrogel preparation step), a step of immersing the preliminary hydrogel in an alcohol solvent and then drying the preliminary hydrogel to obtain a dry preliminary hydrogel (a dry preliminary hydrogel preparation step), and a step of sterilizing the dry preliminary hydrogel until the SAL reaches $1 \times 10^{-6}$ or less (a sterilization step).

(Preliminary hydrogel preparation step)

First, in the preliminary hydrogel preparation step, a preliminary hydrogel including a crosslinked product of a vinyl alcohol polymer is prepared. Specifically, the preliminary hydrogel preparation step preferably includes at least a step of preparing an uncrosslinked polymer solution including the vinyl alcohol polymer (an uncrosslinked polymer solution preparation step), a step of then molding the uncrosslinked polymer solution (a molding step), and a step of then crosslinking the vinyl alcohol polymer contained in the uncrosslinked polymer solution to gel (a crosslinking step). The present invention also provides a method for producing a dry hydrogel including the steps described above. Each of the steps will be specifically described below.

Uncrosslinked polymer solution preparation step

The uncrosslinked polymer solution preparation step is a step of preparing an uncrosslinked polymer solution including the vinyl alcohol polymer, and the uncrosslinked polymer solution can be obtained by dissolving the vinyl alcohol polymer in a solvent. The solvent is preferably water, and may further include a water-soluble organic solvent. Examples of the water-soluble organic solvent include aprotic polar solvents, such as dimethylformamide, dimethylacetamide, dimethylsulfoxide, and N-methylpyrrolidone; monoalcohols, such as methanol, ethanol, propanol, and isopropanol; and polyhydric alcohols, such as ethylene glycol, diethylene glycol, triethylene glycol, and glycerin. The water-soluble organic solvent may be used singly or a mixture of two or more types thereof may be used, or a mixture of the water-soluble organic solvent and water may be used.

When the uncrosslinked polymer solution includes the water-soluble organic solvent, the content thereof is preferably 30 mass% or less, more preferably 20 mass% or less, and still more preferably 10 mass% or less based on the total amount of the uncrosslinked polymer solution. The content of the solvent in the uncrosslinked polymer solution is preferably 50 mass% or more, more preferably 55 mass% or more, and still more preferably 60 mass% or more, and is preferably 99.999 mass% or less, more preferably 99.99 mass% or less, and still more preferably 99.9 mass% or less.

The content of the vinyl alcohol polymer in the uncrosslinked polymer solution is preferably 0.001 mass% or more, more preferably 0.01 mass% or more, and still more preferably 0.1 mass% or more based on the total amount of the uncrosslinked polymer solution. From the viewpoint of suppressing an increase in viscosity of the uncrosslinked polymer solution and obtaining good formability, the content is preferably 50 mass% or less, more preferably 45 mass% or less, and still more preferably 40 mass% or less. When the content of the vinyl alcohol polymer is less than 0.001 mass%, the strength and film thickness of the resulting gel are excessively small, and when the content exceeds 50 mass%, the uncrosslinked polymer solution has a high viscosity, so that it becomes difficult to be molded.

Molding step

The molding step is a step of molding the uncrosslinked polymer solution into a desired shape. The method for molding the uncrosslinked polymer solution is not particularly limited, and the uncrosslinked polymer solution can be molded using a publicly-known method into such a shape as an indefinitely shaped particle, a spherical particle, a finely molded article, an article with any shape molded with a 3D printer, a sheet, a fiber, a hollow fiber, a porous monolith, or a coated article, which are previously mentioned.

Crosslinking step

The crosslinking step is a step of crosslinking the vinyl alcohol polymer after the molding step. The crosslinking in this step can be performed by the method described above. The crosslinking step may be performed with the uncrosslinked polymer solution including a solvent, or may be performed after removing the solvent of the uncrosslinked polymer solution. In the crosslinking step, when crosslinking is performed in the state of an uncrosslinked polymer solution including a solvent, the crosslinked gel severely shrinks in a drying step to be described later, and it may be difficult to form such a shape as a sheet, a fiber, a hollow fiber, or a coating. In this case, it is preferable that the uncrosslinked polymer solution is once dried after molding, and then crosslinked. As the degree of drying of the uncrosslinked polymer solution, a state suitable for the molded article and the crosslinking method can be chosen, but from the viewpoint of easily preventing shrinkage in the drying step, it may be preferable that crosslinking is performed after drying preferably until the solvent content reaches 50% or less, more preferably 25% or less, and still more preferably 10% or less.

The uncrosslinked polymer solution may include not only the vinyl alcohol polymer but also components necessary for crosslinking such as the crosslinking agent and an initiator before being molded. When the uncrosslinked polymer solution is once dried after being molded as described above, components necessary for crosslinking may be incorporated after molding. As described above, optimum crosslinking conditions are chosen depending on the vinyl alcohol polymer to be used and the method of crosslinking, but it is preferable to perform molding before a hydrogel is formed because it becomes difficult to perform molding when the hydrogel is generated after the crosslinking is completed. In some of the crosslinking methods described above, the reaction is started at a temperature equal to or lower than room temperature only by mixing the uncrosslinked polymer solution and a component necessary for crosslinking, and thus, it is desirable to mix them immediately before molding. In the case of crosslinking requiring heat, from the viewpoint of maintaining the activity of the biologically active substance, the temperature of the crosslinking reaction is preferably 100°C or lower, more preferably 60°C or lower, and still more preferably 37°C or lower.

(Conjugation step)

When a biologically active substance is conjugated to the dry hydrogel-forming article of the present invention, the preliminary hydrogel preparation step preferably further includes a conjugation step. The conjugation step is a step of conjugating a biologically active substance to a crosslinked product of a vinyl alcohol polymer to obtain a conjugated hydrogel. When conjugation has been performed in the stage of the uncrosslinked polymer solution, the conjugation step is basically unnecessary. When a carboxylic acid-modified vinyl alcohol-based copolymer or an amino-modified vinyl alcohol polymer is used as the vinyl alcohol polymer, a biologically active substance can be introduced into remaining functional groups by the above-described conjugation method. It is also possible to first introduce a functional group for conjugation into a hydroxy group or the like contained in the crosslinked product of the vinyl alcohol polymer after the crosslinking step, and then perform the conjugation step. As a method for introducing a functional group, a hydroxy group activating reagent, an acid anhydride reagent, an acetal reagent, or the like may be used as described above, and similarly, a biologically active substance can be introduced into a functional group by the above-described conjugation method.

(Dry preliminary hydrogel preparation step)

The hydrogel obtained in the preliminary hydrogel preparation step is dried before sterilization by a general method such as hot air drying, vacuum drying, or freeze drying such that the alcohol solvent content and the water content fall within the following ranges. For example, it is preferable that the preliminary hydrogel obtained in the preliminary hydrogel preparation step is immersed in an alcohol solvent, and then water contained in the hydrogel is replaced with the alcohol solvent and then dried. The temperature of hot air drying is preferably about 30 to 100°C, and more preferably about 37 to 60°C from the viewpoint of avoiding a decrease in activity or loss of activity of a biologically active substance. The drying is preferably performed such that the content of the alcohol solvent in the dry hydrogel-forming article after drying is preferably 0.01 to 15 mass%, more preferably 0.05 to 15 mass%, still more preferably 0.07 to 15 mass%, and further preferably 0.1 to 15 mass%, and the content of water is preferably 0 to 70 mass%, more preferably 0 to 60 mass%, still more preferably 0 to 50 mass%, and further preferably 0 to 40 mass% or less.
The alcohol solvent in which the preliminary hydrogel is to be immersed is not particularly limited, and examples thereof include monoalcohols, such as methanol, ethanol, propanol, and isopropanol, and polyhydric alcohols, such as ethylene glycol, diethylene glycol, triethylene glycol, and glycerin. The alcohol solvent may be used singly or a mixture of two or more types thereof may be used, or a mixture of the alcohol solvent and water may be used.

(Sterilization step)

In the sterilization step, the dry preliminary hydrogel is sterilized until the SAL becomes $1 \times 10^{-6}$ or less. By sterilizing to the above degree, the hydrogel can also be used for applications in which the hydrogel is incorporated into a part of a pharmaceutical product or a medical device used for a human body.
In the present invention, the dry hydrogel-forming article is preferably a dry hydrogel-forming article sterilized in a dried state in which the contents of the alcohol solvent and water are within the above ranges, and is preferably a product sterilized after the drying step. If a conjugated hydrogel before the drying step, that is, containing a large amount of water is sterilized, for example, when a biologically active substance has been conjugated, the activity may decrease. In addition, when the hydrogel is used for cell culture or the like, the adhesion of cells may decrease also due to generation of radicals. Although there is a conventional technique in which sterilization is performed before crosslinking, in the case of sterilization under severe conditions such that the SAL is $1 \times 10^{-6}$ or less, crosslinking of the vinyl alcohol polymer proceeds during the sterilization, and it is considered that sufficient moldability or sufficient strength after molding cannot be obtained.
The sterilization method is not particularly limited as long as SAL = $1 \times 10^{-6}$ or less can be guaranteed. For example, an autoclave sterilization method, an ethylene oxide gas sterilization method, a hydrogen peroxide low-temperature plasma sterilization method, a dry heat sterilization method, a chemical sterilization method using glutaraldehyde or the like, a radiation sterilization method using gamma rays or electron beams, or the like can be used. When a conjugated biologically active substance or the like is conjugated, from the viewpoint of easily maintaining activities thereof and from the viewpoint of enhancing cell adhesiveness when the hydrogel is used for cell culture or the like, it is preferable to employ an ethylene oxide gas sterilization method, a hydrogen peroxide low-temperature plasma sterilization method, or a radiation sterilization method, and it is more preferable to employ a radiation sterilization method from the viewpoint of yielding no residue. The dry hydrogel-forming article of the present invention is preferably free of ethylene oxide gas, glutaraldehyde, and hydrogen peroxide. For sterilization, it is preferable that the preliminary dry hydrogel-forming article is stored in a container, the container is sealed, and then a sterilization step is performed. As the container, a flexible resin container, for example, a pouch made of resin can be used, and sealing can be performed by heat sealing. In addition, a bottle container with a lid that can be opened after sterilization can be used as the container, and sealing can be performed by screwing the lid with a screw or heat sealing like an ampule. For example, in the case of performing radiation sterilization, it is preferable to use a container made of a material that is radiotransparent and retains sufficient strength after irradiation with radiation. Examples of the material of the container include polyolefin resins, such as polyethylene, polypropylene, and polybutylene; copolymers of two or more olefins; polyester resins, such as polyethylene terephthalate and polybutylene terephthalate; vinyl resins, such as polyvinyl alcohol, a saponified product of an ethylene-vinyl acetate copolymer, polyvinyl chloride, and polyvinylidene chloride; and polycarbonate resins, polystyrene resins, silicone resins, polyamide resins, and glass. The container may be composed of a single layer or a laminate of these materials, and it is also possible to use materials obtained by vapor-depositing aluminum, silica, or the like on those materials, a metal foil such as an aluminum film or an aluminum laminate film, or a multilayer body including such a metal foil.

Examples of a method for performing sterilization validation include a half-cycle method, an overkill method, a combined bioburden/BI method, and an absolute bioburden method, any of which can be used. In the case of radiation sterilization, sterilization validation by the absolute bioburden method described in ISO (ISO 11137-2:2013) or JIS (JIS T 0806-2:2014) is performed. Methods of setting a sterilization dose in sterilization validation include Method 1, $VD_{max}$ method, and Method 2, any of which can be used for sterilization in producing the dry hydrogel-forming article of the present invention. Method 1 and the $VD_{max}$ method are methods of setting a sterilization dose based on the number of bacteria attached to the preliminary dry hydrogel-forming article before sterilization, and Method 2 is a method of setting a sterilization dose based on the radiation resistance of bacteria attached to the preliminary dry hydrogel-forming article before sterilization. To determine the sterilization dose by Method 1 and the $VD_{max}$ method, the bioburden test described in ISO 11737-1:2006 or JIS T11737-1:2013 is performed, and the number of bacteria attached to the preliminary dry hydrogel-forming article before sterilization can be measured. Method 1 and the $VD_{max}$ method are more preferable in consideration of the number of necessary samples and cost, and the $VD_{max}$ method is still more preferable in consideration of cost reduction.

To achieve SAL = $1 \times 10^{-6}$ or less, an irradiation dose of 11k gray (Gy) or more is required in Method 1, and an irradiation dose of 15 kGy or more is required in the $VD_{max}$ method. Method 2 requires an irradiation dose of 8.2 kGy or more. Conventionally, it has been recognized that a hydrogel (particularly a conjugated hydrogel) cannot withstand the gamma ray irradiation dose of such a very high energy. In one preferred embodiment of the present invention, by performing sterilization on a dried preliminary dry hydrogel-forming article, it is possible to prevent damage to the product even by gamma irradiation with a high energy of, for example, 8.2 kGy or more, suppress a decrease in cell adhesiveness, and, when a biologically active substance is conjugated, maintain the activity thereof in a desired range. In one preferred embodiment of the production method of the present invention, it is preferable to perform radiation sterilization in the step of sterilizing until the SAL becomes $1 \times 10^{-6}$ or less, and it is more preferable that the radiation dose in the radiation sterilization is 8.2 kGy or more.


3. Hydrogel


The dry hydrogel-forming article of the present invention can be swollen in water and then used as a hydrogel. The present invention also provides a hydrogel that is a swollen product (preferably a water-swollen product) of the dry hydrogel-forming article of the present invention. The hydrogel of the present invention usually includes water, and may include a water-soluble organic solvent other than water, or may include a mixture of water and a water-soluble organic solvent. As the water-soluble organic solvent, water-soluble organic solvents such as aprotic polar solvents, such as dimethylformamide, dimethylacetamide, dimethylsulfoxide, and N-methylpyrrolidone; monoalcohols, such as methanol, ethanol, propanol, and isopropanol; and polyhydric alcohols, such as ethylene glycol, diethylene glycol, triethylene glycol, or glycerin may be mixed and then used. The content of the solvent (preferably water and/or a water-soluble organic solvent) in the hydrogel is preferably 30 to 99.9 mass%, more preferably 40 to 99 mass%, and still more preferably 50 to 97 mass% based on the total amount of the hydrogel. The content of water in the hydrogel is preferably 30 to 95 mass%, more preferably 40 to 90 mass%, and still more preferably 50 to 90 mass% based on the total amount of the hydrogel. The content of the water-soluble organic solvent in the hydrogel is preferably 30 to 95 mass%, more preferably 40 to 95 mass%, and still more preferably 50 to 95 mass%.

From the viewpoint of strength, the solid content in the hydrogel of the present invention is preferably 0.1 to 70 mass%, more preferably 1 to 60 mass%, and still more preferably 3 to 50 mass% based on the total amount of the hydrogel. The solid content of the hydrogel can be calculated from the following Formula (IV) from the weight of the hydrogel before drying and, for example, the weight of the hydrogel after drying the hydrogel at 120°C for 5 hours.

Solid content [mass%] = (weight of hydrogel after drying/weight of hydrogel before drying) $\times$ 100          (IV)

The hydrogel of the present invention may be used in combination with cells or may contain cells. When used in combination with cells or containing cells, it is preferable to supply water to the article to form a hydrogel. The term "cell" as referred to in the present description is not particularly limited, and preferably includes pluripotent stem cells, tissue stem cells, somatic cells, established cells derived from mammals to be used for production of useful substances such as pharmaceuticals, therapies, etc., and insect cells.

Cells include adherent cells and suspension cells. The adherent cell refers to a cell that proliferates by adhering to a carrier such as the hydrogel of the present invention during cell culture. The suspension cell refers to a cell that does not basically require adhering to a carrier in cell proliferation. The suspension cell includes a cell capable of weakly adhering to a carrier.

The pluripotent stem cells are stem cells having the ability to differentiate into cells of any tissue (differentiation pluripotency), and examples thereof include embryonic stem cells (ES cells), induced pluripotent stem cells (iPS cells), embryonic germ stem cells (EG cells), and germ stem cells (GS cells).

The tissue stem cell means a stem cell having an ability to differentiate into various cell types (pluripotency) although the tissue to be differentiated is limited, and examples of the tissue stem cell include bone marrow undifferentiated mesenchymal stem cells, skeletal muscle stem cells, hematopoietic stem cells, neural stem cells, liver stem cells, adipose tissue stem cells, epidermal stem cells, intestinal stem cells, sperm stem cells, pancreatic stem cells (e.g., pancreatic ductal epithelial stem cells), leukocyte stem cells, lymphocyte stem cells, corneal stem cells.

The somatic cell refers to a cell constituting a multicellular organism, and examples thereof include, but are not limited to, osteoblasts, chondrocytes, hematopoietic cells, epithelial cells (e.g., mammary epithelial cells), endothelial cells (e.g., vascular endothelial cells), epidermal cells, fibroblasts, mesenchyme-derived cells, cardiomyocytes, myoblasts, smooth muscle cells, living body-derived skeletal muscle cells, human tumor cells, fibrous cells, EB virus mutant cells, hepatocytes, renal cells, bone marrow cells, macrophages, hepatocytes, small intestinal cells, mammary cells, salivary gland cells, thyroid cells, skin cells, plasma cells, T cells, B cells, killer cells, lymphoblasts, and pancreatic $\beta$ cells.

Examples of the established cells derived from mammals include CRFK cells, 3T3 cells, A549 cells, AH130 cells, B95-8 cells, BHK cells, BOSC23 cells, BS-C-1 cells, C3H10T1/2 cells, C-6 cells, CHO cells, COS cells, CV-1 cells, F9 cells, FL cells, FL5-1 cells, FM3A cells, G-361 cells, GP+E-86 cells, GP+envAm12 cells, H4-II-E cells, HEK293 cells, HeLa cells, HEp-2 cells, HL-60 cells, HTC cells, HUVEC cells, IMR-32 cells, IMR-90 cells, K562 cells, KB cells, L cells, L5178Y cells, L-929 cells, MA104 cells, MDBK cells, MDCK cells, MIA PaCG-2 cells, N18 cells, Namalwa cells, NG108-15 cells, NRK cells, OC10 cells, OTT6050 cells, P388 cells, PA12 cells, PA317 cells, PC-12 cells, PER.C6 cells, PG13 cells, QGH cells, Raji cells, RPMI-1788 cells, SGE1 cells, Sp2/O-Ag14 cells, ST2 cells, THP-1 cells, U-937 cells, V79 cells, VERO cells, WI-38 cells, $\psi$2 cells, and $\psi$CRE cells.

Examples of the insect cells include silkworm cells (e.g., BmN cells and BoMo cells), Bombyx mandarina cells, tussah cells, ailanthus moth cells, cabbage armyworm cells (e.g., Sf9 cells and Sf21 cells), Lemyra imparilis cells, leaf roller cells, vinegar fly cells, Boettcherisca peregrine cells, Aedes albopictus cells, Papilio xuthus cells, American cockroach cells, and Trichoplusia ni cells (e.g., Tn-5 cells, High Five cells and MG1 cells).

The cells may be aggregated together or may be differentiated. The aggregated cells may have a function as an organ. The cells may be those immediately after being collected from a living body or may be those cultured. Cells collected from a living body may form an organ.

When the hydrogel of the present invention is used, for example, for culturing cells in contact with the cells, it is preferable to add a medium component. The medium to be used is not particularly limited, and a medium suitable for cells can be freely selected and used. As the culture medium, basic culture media such as DMEM, MEM, or F12 to which fetal bovine serum is added, serum-reduced culture media, serum-free culture media, and the like can be used. Examples of the serum-free medium that can be used include heterologous component-free (xeno-free) media and completely synthetic media that are free of not only serum but also protein components and animal components.

The hydrogel of the present invention is a sufficiently sterilized hydrogel, and has good dispersibility because cohesion at the time of drying is suppressed. In addition, contamination of hydrogel fragments or the like is prevented. The hydrogel of the present invention preferably has characteristics such as maintaining a high degree of swelling, having high strength, maintaining the activity of a biologically active substance or the like when containing the substance or the like, and having high adhesiveness when used for cell adhesion. The hydrogel of the present invention preferably can provide a sterilized hydrogel superior in hydrophilicity, reactivity, biodegradability, biocompatibility, low toxicity, and the like, and having high flexibility and strength without using an expensive process such as a sterile environment. Therefore, the hydrogel of the present invention is useful as a carrier or support. A carrier formed of the hydrogel of the present invention can be suitably used for such applications as an enzyme-immobilized carrier, an affinity carrier, a cell culture carrier, and a drug delivery carrier.

The hydrogel of the present invention is a hydrogel obtained by swelling the dry hydrogel-forming article of the present invention, and the vinyl alcohol polymer included in the dry hydrogel-forming article of the present invention is similarly included in the hydrogel of the present invention. Unless explicitly denied in the present description, the details, production method, use method, and the like of the hydrogel of the present invention are the same as those described in "1. Dry hydrogel-forming article".

The hydrogel of the present invention is a hydrogel formed via a process in which the dry hydrogel-forming article of the present invention comes into contact with an aqueous solution such as water, a buffer solution, a body fluid, or a culture solution to swell. The hydrogel of the present invention may be a hydrogel that is completely swollen until the dry hydrogel-forming article no longer swells, or may be a hydrogel that can further swell and still have hydrogel-forming properties.

The shape of the hydrogel is not particularly limited, and similarly to the dry hydrogel-forming article, the hydrogel may be an indefinitely shaped particle, a spherical particle, a finely molded article, an article with any shape formed with a 3D printer, a sheet, a fiber, a hollow fiber, a porous monolith, or a coated article. The hydrogel of the present invention is preferably a spherical particle, and more preferably a spherical particle having a particle size of 10 to 5000 μm.

In the present description, the "amide linkage-conjugated hydrogel" means a hydrogel in which a biologically active substance having an amino group is covalently bound to a crosslinked product of a vinyl alcohol polymer having ethylenically unsaturated groups and carboxy groups by an amide linkage at a carboxy group of the vinyl alcohol polymer.

Furthermore, the form, dimensions, production method, and the like of the hydrogel may be those described above in "1. Dry hydrogel-forming article".

The hydrogel of the present invention is a swollen product of the dry hydrogel-forming article of the present invention, and preferably a water-swollen product of the dry hydrogel-forming article of the present invention. The water-swollen product is a swollen product swollen by being brought into contact with a solution containing at least water, and may be, for example, a swollen product swollen by being brought into contact with water, or a swollen product swollen by being brought into contact with an aqueous solution such as a buffer solution, a body fluid, or a culture solution. The alcohol solvent having been included in the dry hydrogel-forming article of the present invention may or may not be included in the hydrogel of the present invention. For example, when the dry hydrogel-forming article of the present invention including a crosslinked product of a vinyl alcohol polymer and an alcohol solvent is put in water to swell, the alcohol solvent included in the dry hydrogel-forming article of the present invention may be released into the water, and the hydrogel of the present invention may no longer include alcohol. Such a hydrogel can also be said to be a water-swollen product of the dry hydrogel-forming article of the present invention. Since the hydrogel of the present invention is a swollen product of a dry hydrogel-forming article that satisfies a specific degree of cohesion and sterility assurance level SAL, the hydrogel contains less contaminants such as gel fragments and is a sufficiently sterilized hydrogel.

The sterility assurance level SAL in the hydrogel of the present invention is not particularly limited, and the hydrogel preferably has a sterility assurance level SAL = $1 \times 10^{-6}$ or less, which is equivalent to that of the dry hydrogel-forming article.

<Crosslinked product of vinyl alcohol polymer>

The hydrogel of the present invention includes a crosslinked product of a vinyl alcohol polymer similar to that of the dry hydrogel-forming article of the present invention. Unless explicitly denied in the present description, regarding the crosslinked product of the vinyl alcohol polymer included in the hydrogel, the description regarding the crosslinked product of the vinyl alcohol polymer included in the dry hydrogel-forming article similarly applies.

<Biologically active substance>

When the dry hydrogel-forming article of the present invention further includes a biologically active substance, the hydrogel of the present invention also includes the biologically active substance. Unless explicitly denied in the present description, regarding the biologically active substance that may be included in the hydrogel, the description regarding the biologically active substance that may be included in the dry hydrogel-forming article similarly applies.

<Method for producing hydrogel>

The hydrogel of the present invention is a swollen product of the dry hydrogel-forming article of the present invention, and is obtained by bringing the dry hydrogel-forming article into contact with water, the above-described water-soluble organic solvent, an aqueous solution, a mixture thereof, or the like.

The hydrogel of the present invention is a sufficiently sterilized hydrogel, in which contamination of fragments or breakage of a surface has not occurred because cohesion during drying is suppressed, and the hydrogel is suitably used as a hydrogel to be used for medical use or the like. In one preferred embodiment, the hydrogel of the present invention also has advantages such as being superior in hydrophilicity, reactivity, biodegradability, and biocompatibility, being high in flexibility and strength, and having low toxicity because of use of a crosslinking method with low toxicity, and having efficient introduction of a biologically active substance or enzyme. Therefore, the hydrogel of the present invention is useful as a carrier or support. A carrier including the hydrogel of the present invention can be suitably used for such applications as an enzyme-immobilized carrier, an affinity carrier, a cell culture carrier, and a drug delivery carrier. With the dry hydrogel-forming article of the present invention, an excellent hydrogel as described above can be provided.

Examples

Hereinafter, the present invention will be described in more detail with reference to Examples, but the present invention is not limited by these Examples.

[Raw materials used]

Main components used in Synthesis Examples, Examples and Comparative Examples are shown below.

<Raw material PVA>

· PVA117: polyvinyl alcohol (trade name: "PVA117", degree of polymerization: 1700; degree of saponification: about 98.0 to 99.0 mol%, viscosity (4%, 20°C): 25.0 to 31.0 mPa·s, manufactured by Kuraray Co., Ltd.)
The degree of polymerization of each raw material PVA was measured in accordance with JIS K 6726:1994.
· AF-17: polyvinyl alcohol (trade name: "AF-17", 1.3 mol% itaconic acid copolymerization (Scientific Reports, 2017, Vol. 7, p. 45146), degree of polymerization: 1700, degree of saponification: 96.5 mol% or more, viscosity (4%, 20°C): 30 ± 3 mPa·s, manufactured by JAPAN VAM & POVAL CO., LTD.)

<Ethylenically unsaturated group-containing compound>

· Vinyl methacrylate: manufactured by Tokyo Chemical Industry Co., Ltd.
· 5-Norbornene-2-carboxaldehyde: manufactured by Tokyo Chemical Industry Co., Ltd.

<Synthesis reagent>

· 50% Glutaraldehyde solution: manufactured by FUJIFILM Wako Pure Chemical Corporation
· Triethylamine: manufactured by FUJIFILM Wako Pure Chemical Corporation
· Succinic anhydride: manufactured by FUJIFILM Wako Pure Chemical Corporation
· Liquid paraffin: manufactured by FUJIFILM Wako Pure Chemical Corporation
· Span80: sorbitan monooleate, manufactured by FUJIFILM Wako Pure Chemical Corporation

<Radical polymerization initiator>

· Potassium persulfate: manufactured by FUJIFILM Wako Pure Chemical Corporation
· L0290: lithium phenyl(2,4,6-trimethylbenzoyl)phosphinate photoradical polymerization initiator, trade name: "L0290", manufactured by Tokyo Chemical Industry Co., Ltd.)

<Polythiol>

· 3,6-Dioxa-1,8-octanedithiol: manufactured by Tokyo Chemical Industry Co., Ltd.

<Activation reagent for hydroxy group>

· 1,1'-Carbonyldiimidazole: manufactured by Tokyo Chemical Industry Co., Ltd.
· Succinic anhydride: manufactured by Tokyo Chemical Industry Co., Ltd., or manufactured by FUJIFILM Wako Pure Chemical Corporation

<Conjugating reagent>

· 1-(3-Dimethylaminopropyl)-3-ethylcarbodiimide hydrochloride: manufactured by FUJIFILM Wako Pure Chemical Corporation
· N-Hydroxysuccinimide: manufactured by Tokyo Chemical Industry Co., Ltd.

<Biologically active substance>

· Gelatin (derived from porcine, type A): manufactured by Sigma-Aldrich Japan
· Collagen (trade name: "Cellmatrix", type I-C): manufactured by Nitta Gelatin Inc.

<Solvent>

· Ion-exchanged water: ion-exchanged water having an electrical conductivity of $0.08 \times 10^{-4}$ S/m or less
· PBS: prepared by dissolving PBS tablet (manufactured by Takara Bio Inc.) in a prescribed amount of ion-exchanged water.
· MES buffer: A 0.1 mol/L aqueous solution of 2-morpholinoethanesulfonic acid monohydrate (manufactured by DOJINDO LABORATORIES) was prepared, and neutralized with a 0.1 mol/L aqueous NaOH solution to have a pH of 5.6.

[Method for measuring compound synthesized in Synthesis Example]

<Introduction rate of ethylenically unsaturated groups and introduction rate of carboxy groups (succinic acid)>

The introduction rates of ethylenically unsaturated groups and carboxy groups (succinic acid) of the vinyl alcohol polymers having ethylenically unsaturated groups obtained in the following Synthesis Examples was measured by $^1$H-NMR. The introduction rates are determined from the ratio of the integral value of the signal of ethylenically unsaturated groups or carboxy groups (methylene succinate groups) to the integral value of the signal of the vinyl alcohol polymer. Similarly, a hydrogel particle and a hydrogel sheet can also be subjected to $^1$H-NMR measurement in a state of being swollen in a deuterated solvent, and the introduction rates in the hydrogel particle and the hydrogel sheet are determined from the ratio of the integral value of the signal of carboxy groups (methylene succinate groups) to the integral value of the signal of the vinyl alcohol polymer.

[$^1$H-NMR measurement conditions]

Instrument: nuclear magnetic resonance spectrometer "JNM-ECX400" manufactured by JEOL Ltd.
Temperature: 25°C

<Water content and content of alcohol solvent>

The water content of the dry hydrogel-forming articles obtained in the following Examples was measured with a heat-dry moisture analyzer when only water was contained as a solvent, or by $^1$H-NMR when a water-soluble organic solvent such as an alcohol solvent was contained.

[Conditions for measurement with heat-dry moisture analyzer]

Instrument: Heat-dry moisture analyzer manufactured by A&D Co., Ltd.

[$^1$H-NMR measurement conditions]

The contents of water and the alcohol solvent can be calculated by $^1$H-NMR (deuterated DMSO solvent). The water content is determined from the ratio of the integral value of the signal of water (3.3 ppm) and the integral value of the signal of the vinyl alcohol polymer. The water originally contained in the deuterated DMSO solvent is subtracted.

Instrument: nuclear magnetic resonance spectrometer "JNM-ECX400" manufactured by JEOL Ltd.
Temperature: 25°C

<Sterility>

By performing gamma ray sterilization at a sterilization dose determined by the $VD_{max}$ method, it was ensured that the dry hydrogel-forming article achieved sterility assurance level SAL = $10^{-6}$ or less. When the sterility assurance level SAL = $10^{-6}$ or less was achieved, the sterility was evaluated as "∘", and when it was not achieved, the sterility was evaluated as "×".

<Degree of cohesion>

The degree of cohesion of the dry hydrogel-forming article is determined by taking 0.2 g of dry hydrogel-forming articles, separating, by visual observation, cohered materials in which two or more of the dry hydrogel-forming articles were cohered, measuring the weight of the cohered materials separated, and calculating the degree of cohesion from the following formula (II). When the degree of cohesion is 10% or less, the cohesiveness is evaluated as "∘", and when it exceeds 10%, the cohesiveness is evaluated as "×".

$$\text{Degree of cohesion [\%]} = (\text{weight of cohered material/total weight}) \times 100 \cdots$$

$$(II)$$

<Breakage rate>

Regarding the breakage rate of a dry hydrogel-forming article, 0.1 g of the dry hydrogel-forming article was weighed and put into a 50 ml glass vial (bottom diameter: 25 mm) together with 10 ml of ion-exchanged water, and the breakage state after stirring at a speed of 400 rpm for 3 hours with a stirrer chip having a diameter of 20 mm was evaluated. That is, the state of a solid component after stirring is observed with a microscope, the solid component is observed in a state where the solid component can be seen in 60% or more of the visual field, the damaged portion and the non-damaged portion are visually discriminated, and the breakage rate is calculated from the ratio of the area of the damaged portion to the area of the entire solid component. In the present example and the comparative example, since the breakage rate was very high or very low, the ratio of the areas was calculated by visual observation, but the above areas may be measured using image software to calculate the breakage rate.

[Synthesis Examples]

<Synthesis of vinyl alcohol polymer having ethylenically unsaturated group>

[Synthesis Example 1-1]

40 g (monomer repeating unit: 908 mmol) of PVA117 (raw material PVA) was placed in a separable flask equipped with a 1-L Dimroth condenser. 350 mL of dimethylsulfoxide (DMSO) was added, and stirring with a mechanical stirrer was started. The temperature was raised to 80°C by a water bath, and then stirring was continued at 80°C for 4 hours. After visually confirming that the raw material PVA was dissolved, 2.1 g (18.7 mmol) of vinyl methacrylate was added and stirred with heating at 80°C, and the mixture was further stirred at 80°C for 3 hours. After being allowed to cool, the reaction solution was poured into 2 L of methanol with stirring. The stirring was stopped and the mixture was left to stand for 1 hour. The solid obtained was collected, and then further immersed in 1 L of methanol for 1 hour and thereby washed. This washing operation was performed three times in total. The collected solid was vacuum-dried at room temperature overnight, affording methacryloylated PVA117. The introduction rate of an ethylenically unsaturated group (methacryloyl group) of the methacryloylated PVA117 was 2.0 mol% based on the repeating units of the raw material PVA (the methacryloylated PVA117 is hereinafter abbreviated as "MA-PVA117(2.0)").

[Synthesis Example 1-2]

60 g (monomer repeating unit: 1.36 mol) of PVA117 (raw material PVA) was placed in a separable flask equipped with a 1-L Dimroth condenser. 540 mL of ion-exchanged water was added, and stirring with a mechanical stirrer was started. The temperature was raised to 80°C by a water bath, and then stirring was continued at 80°C for 4 hours. After visually confirming that the raw material PVA was dissolved, the temperature was lowered to 40°C. While the solution was stirred at 40°C, 2.5 g (20.5 mmol) of 5-norbornene-2-carboxyaldehyde and 22 mL of a 10 vol% aqueous sulfuric acid solution were added, and the mixture was further stirred at 40°C for 4 hours. The mixture was allowed to cool, and then 80 mL of a 1 N aqueous NaOH solution was added for neutralization. The resulting mixture was placed in a dialysis membrane with a molecular weight cutoff of 3,500, and desalted (performed 4 times with 5 L of ion-exchanged water). The desalted aqueous solution was poured into 2 L of methanol with stirring, and the mixture was left to stand for 1 hour. The solid obtained was collected, and then further immersed in 1 L of methanol for 1 hour and thereby washed. The collected solid was vacuum-dried at room temperature overnight, affording norbornene-introduced (Nor) PVA117. The introduction rate of an ethylenically unsaturated group (norbornenyl group) of the norbornene-introduced PVA117 was 1.3 mol% based on the repeating units of the raw material PVA (the norbornene-introduced PVA117 is hereinafter abbreviated as "Nor-PVA117(1.3)").

[Synthesis Example 1-3]

10 g (monomer repeating unit: 220 mmol) of MA-PVA117(2.0) prepared in Synthesis Example 1-1 was placed in a separable flask equipped with a 0.5-L Dimroth condenser. 90 mL of dimethylsulfoxide (DMSO) was added, and stirring with a mechanical stirrer was started. The temperature was raised to 80°C by a water bath, and then stirring was continued at 80°C for 4 hours. After visually confirming that the raw material PVA was dissolved, the water bath was set to 60°C. After confirming that the internal temperature reached 60°C, 1.2 g (12.1 mmol) of triethylamine and 1.1 g (11 mmol) of succinic anhydride were added, and the mixture was further stirred at 60°C for 5 hours. After being allowed to cool, the reaction solution was poured into 0.5 L of methanol with stirring. The stirring was stopped and the mixture was left to stand for 1 hour. The solid obtained was collected, and then immersed in 0.5 L of methanol for 1 hour and thereby washed. This washing operation was performed three times in total. The collected solid was vacuum-dried at room temperature overnight, affording methacryloylated PVA117 with succinic acid introduced thereinto. The introduction rate of succinic acid (SA) was 3.4 mol% based on the repeating units of MA-PVA117(2.0) (the methacryloylated PVA117 is hereinafter abbreviated as "MA-PVA117(2.0)-SA(3.4)").

<Production of molded article (particle, sheet)>

[Synthesis Example 1-A: Particle]

440 mL of ion-exchanged water was added to 60 g of MA-PVA117(2.0), and the mixture was dissolved at 80°C for 4 hours with stirring. After cooling to room temperature, potassium persulfate as a water-soluble thermal radical polymerization initiator was added to and dissolved in the aqueous solution of MA-PVA117(2.0) to attain a concentration of 0.1 mass%, whereby an uncrosslinked polymer solution was prepared. A separable flask equipped with a 5-L Dimroth condenser was charged with 3300 mL of liquid paraffin and 8 g of Span80, and to this solution was slowly added the uncrosslinked polymer solution. This mixed liquid was made into a W/O dispersion liquid while being stirred with a mechanical stirrer at 350 rpm, and the temperature was raised to 40°C by a water bath and then purged with nitrogen for 30 minutes. Thereafter, stirring was continued at 70°C for 3 hours. The mixture was cooled to room temperature, and the liquid paraffin in which hydrogel particles were dispersed was filtered through a mesh with an opening size of 100 μm. The hydrogel particles obtained were washed with 3 L in total of hexane to remove liquid paraffin, and the hydrogel particles obtained were classified into particle sizes of 180 to 300 μm using JIS standard sieves. Further, the hydrogel particles were charged into 1 L of acetone, dehydrated, and then dried under reduced pressure, affording dry hydrogel particles (hereinafter abbreviated as "MA-PVA117(2.0) gel particles").

[Synthesis Example 1-B: Particle]

440 mL of ion-exchanged water was added to 60 g of Nor-PVA117(1.3), and the mixture was dissolved at 80°C for 4 hours with stirring. After cooling to room temperature, 3.4 g of 3,6-dioxa-1,8-octanedithiol as a polythiol was added to the aqueous solution of Nor-PVA117(1.3), and the mixture was stirred. Potassium persulfate as a water-soluble thermal radical polymerization initiator was added to and dissolved in this solution to attain a concentration of 0.1 mass%, whereby an uncrosslinked polymer solution was prepared. Using this uncrosslinked polymer solution, dry hydrogel particles were obtained in the same manner as in Synthesis Example 1-A (the dry hydrogel particles are hereinafter abbreviated as "Nor-PVA117(1.3) gel particles").

[Synthesis Example 1-C: Sheet]

44 mL of ion-exchanged water was added to 6 g of MA-PVA117(2.0)-SA(3.4), and the mixture was dissolved at 80°C for 4 hours with stirring. After cooling to room temperature, potassium persulfate as a water-soluble thermal radical polymerization initiator was added to and dissolved in the aqueous solution of MA-PVA117(2.0)-SA(3.4) to attain a concentration of 0.1 mass%, whereby an uncrosslinked polymer solution was prepared. This uncrosslinked polymer solution was poured between two glass plates with a 0.5 mm spacer interposed therebetween under nitrogen, and cured at 40°C for 3 hours. The cured hydrogel sheet ($10 \times 20$ cm) having a thickness of 0.5 mm was washed with 1.5 L in total of ion-exchanged water. Further, the hydrogel sheet was immersed in 0.5 L of acetone to be dehydrated, and dried under reduced pressure, affording a dry hydrogel sheet (hereinafter abbreviated as "MA-PVA117(2.0)-SA(3.4) gel sheet"). The introduction rate of succinic acid (SA) (active group introduction rate) of the dry PVA sheet was 3.4 mol%.

[Synthesis Example 1-D: Sheet]

44 mL of ion-exchanged water was added to 6 g of PVA117, and the mixture was dissolved at 80°C for 4 hours with stirring. After cooling to room temperature, 7.5 mL of a 50% glutaraldehyde (GA) solution was added to the aqueous PVA117 solution and mixed, and then 6 mL of a 1 mol/L aqueous hydrochloric acid solution was further added and mixed quickly to prepare an uncrosslinked polymer solution. This uncrosslinked polymer solution was poured between two glass plates with a 0.5 mm spacer interposed therebetween, and cured at 65°C for 7 hours. The cured hydrogel sheet ($10 \times 20$ cm) having a thickness of 0.5 mm was washed with 1.5 L in total of ion-exchanged water. Further, the hydrogel sheet was immersed in 0.5 L of acetone to be dehydrated, and dried under reduced pressure, affording a dry hydrogel sheet (hereinafter abbreviated as "GA-crosslinked PVA117 gel sheet").

<Activation of hydroxy group>

[Synthesis Example 1-i: Introduction of succinic acid into molded article (particle)]

1.5 g of the MA-PVA117(2.0) gel particles (monomer repeating unit: 33 mmol) prepared in Synthesis Example 1-A was placed in a separable flask equipped with a 0.5-L Dimroth condenser. 100 mL of dimethylsulfoxide (DMSO) was added and stirring with a mechanical stirrer, whereby the gel particles were swollen. The mixture was stirred in a water bath at 60°C for 1 hour, then 364 mg (3.6 mmol) of triethylamine and 330 mg (3.3 mmol) of succinic anhydride were added, and the mixture was further stirred at 60°C for 5 hours. After being allowed to cool, the particles were collected by filtration and washed twice with 100 mL of DMSO and twice with 100 mL of ion-exchanged water. Next, the particles were immersed three times in 100 mL of acetone to be dehydrated, and the particles obtained were dried under reduced pressure, affording dry hydrogel particles into which succinic acid (SA) had been introduced. The introduction rate of succinic acid (active group introduction rate) was 10.9 mol% based on the repeating units of MA-PVA117(2.0) (the resulting dry hydrogel particles are hereinafter abbreviated as "SA-introduced-MA-PVA117(2.0) gel particles").

[Synthesis Example 1-ii: Introduction of succinic acid into molded article (particle)]

Dry hydrogel particles into which succinic acid had been introduced were obtained in the same manner as in Synthesis Example 1-i except that the dry Nor-PVA117(1.3) gel particles prepared in Synthesis Example 1-B were used. The introduction rate of succinic acid (SA) (active group introduction rate) was 8.7 mol% based on the repeating units of Nor-PVA117(1.3) (the resulting dry hydrogel particles are hereinafter abbreviated as "SA-introduced-Nor-PVA117(1.3) gel particles").

[Synthesis Example 1-iii: Introduction of succinic acid to molded article (sheet)]

A dry hydrogel sheet into which succinic acid was introduced was obtained in the same manner as in Synthesis Example 1-i except that the dry GA crosslinked PVA117 gel sheet prepared in Synthesis Example 1-D was used. The introduction rate of succinic acid (SA) (active group introduction rate) was 9.8 mol% based on the repeating units of the GA-crosslinked PVA117 (the dry hydrogel sheet is hereinafter abbreviated as "SA-introduced-GA-crosslinked PVA117 gel sheet").

[Synthesis Example 1-iv: Introduction of carbonyl imidazolyl groups to molded article (particle)]

377 mg (2.3 mmol) of carbonyldiimidazole (hereinafter abbreviated as "CDI") was dissolved in 7 g of dry acetonitrile, and to this was added 1.0 g of the dry MA-PVA117(2.0) gel particles (monomer repeat unit: 23 mmol) prepared in Synthesis Example 1-A. The mixture was made to react in a centrifuge tube at 40°C for 4 hours while the centrifuge tube was shaken, and then washed 4 times with 50 mL of dry acetone. The particles washed with acetone were vacuum-dried at room temperature overnight, affording carbonylimidazolylated (CI-introduced) hydrogel particles (hereinafter abbreviated as "CI-introduced-MA-PVA117(2.0) gel particles"). The introduction rate of carbonylimidazolyl groups (active group introduction rate) was 13.5% based on the repeating units of MA-PVA117(2.0).

<Conjugation>

[Synthesis Example 1-a]

1 g of the dry SA-introduced-MA-PVA117(2.0) gel particles (succinic acid introduction amount: about 1.9 mmol) prepared in Synthesis Example 1-i were swollen in MES buffer (pH = 5.6) at room temperature overnight. The swollen gel particles were dispersed in 45 mL of MES buffer, 0.78 g (6.8 mmol) of N-hydroxysuccinimide and 0.65 g (3.4 mmol) of 1-(3-dimethylaminopropyl)-3-ethylcarbodiimide hydrochloride were added thereto with stirring using a magnetic stirrer, and the mixture was shaken at room temperature for 1 hour. The operation of washing the gel particles with 30 mL of MES buffer for 10 minutes was repeated three times, and the gel particles were added to 70 mL of a 1 mg/mL gelatin PBS solution. The operation of shaking the mixture at room temperature for 3 hours and washing the resulting hydrogel particles with 70 mL of ion-exchanged water (heated to 60°C) for 20 minutes was repeated twice, affording gelatin-conjugated hydrogel particles (hereinafter abbreviated as "gelatin-SA-introduced-MA-PVA117(2.0) gel particles"). Some of the gelatin-SA-introduced-MA-PVA117(2.0) gel particles were immersed in excess PBS overnight and the amount of immobilized gelatin per gel weight (100 mg) was measured by the bicinchoninic acid (BCA) method (BCA Protein Assay Kit (manufactured by Takara Bio Inc.)). The conjugation density of the biologically active substance was 48.5 μg/100 mg gel particles.

[Synthesis Example 1-b]

Collagen-conjugated hydrogel particles were obtained in the same manner as in Synthesis Example 1-a except that collagen was used instead of gelatin (the collagen-conjugated hydrogel particles are hereinafter abbreviated as "collagen-SA-introduced-MA-PVA117(2.0) gel particles"). The amount of immobilized collagen (the conjugation density of biologically active substance) of the collagen-SA-introduced-MA-PVA117(2.0) gel particles was also measured in the same manner as in Synthesis Example a.

[Synthesis Example 1-c]

Gelatin-conjugated hydrogel particles were obtained in the same manner as in Synthesis Example 1-a except that 1 g of the dry SA-introduced-Nor-PVA117(1.3) gel particles prepared in Synthesis Example 1-ii were used (the gelatin-conjugated hydrogel particles are hereinafter abbreviated as "gelatin-SA-introduced-Nor-PVA117(1.3) gel particles"). The amount of immobilized gelatin (the conjugation density of biologically active substance) of the gelatin-SA-introduced-Nor-PVA117(2.0) gel particles was also measured in the same manner as in Synthesis Example 1-a.

[Synthesis Example 1-d]

A gelatin-conjugated hydrogel sheet was obtained in the same manner as in Synthesis Example 1-a except that 1 g of the dry MA-PVA117(2.0)-SA(3.4) gel sheet prepared in Synthesis Example 1-C was used (the gelatin-conjugated hydrogel sheet is hereinafter abbreviated as a "gelatin-MA-PVA117(2.0)-SA(3.4) gel sheet"). The amount of immobilized gelatin (the conjugation density of biologically active substance) of the gelatin-MA-PVA117(2.0)-SA(3.4) gel sheet was also measured in the same manner as in Synthesis Example 1-a.

[Synthesis Example 1-e]

A gelatin-conjugated hydrogel sheet was obtained in the same manner as in Synthesis Example 1-a except that 1 g of the dry SA-introduced-GA-crosslinked PVA117 gel sheet prepared in Synthesis Example 1-iii was used (the gelatin-conjugated hydrogel sheet is hereinafter abbreviated as "gelatin-SA-introduced-GA-crosslinked PVA117 gel sheet"). The amount of immobilized gelatin (the conjugation density of biologically active substance) of the gelatin-SA-introduced-GA-crosslinked PVA117 gel sheet was also measured in the same manner as in Synthesis Example 1-a.

[Synthesis Example 1-f]

0.6 g of gelatin was dissolved in 100 mL of PBS at 60°C under stirring with a magnetic stirrer. This gelatin solution was cooled to room temperature, and the CI-introduced-MA-PVA117(2.0) gel particles prepared in Synthesis Example 1-iv were added thereto. The mixture was shaken and stirred overnight at room temperature, and washed three times with 100 mL of PBS, affording gelatinized gel particles (hereinafter abbreviated as "gelatin-urethane linkage-MA-PVA117(2.0) gel particles"). The amount of immobilized gelatin (the conjugation density of biologically active substance) of the gelatin-urethane linkage-MA-PVA117(2.0) gel particles was measured in the same manner as in Synthesis Example 1-a.

<Drying and gamma ray sterilization of particles>

[Example 1-1]

Immersion of 2 g of the gelatin-SA-introduced-MA-PVA117(2.0) gel particles (swollen with ion-exchanged water) prepared in Synthesis Example 1-a in 25 mL of ethanol was repeated three times, and the dehydrated conjugated gel particles were vacuum-dried at room temperature overnight. The water content and the content of the alcohol solvent of the resulting dry particles were calculated by $^1$H-NMR (deuterated DMSO solvent) in accordance with the measurement method described above. Gamma-ray irradiation was performed in the same manner as in Comparative Example 1-1 described later, affording a sterilized amide linkage-conjugated hydrogel. The measurement results of the water content and the content of the alcohol solvent and the evaluation results of the sterility and the cohesiveness are shown in Table 1. The degree of cohesion and the breakage rate of the dry hydrogel-forming article obtained in Example 1-1 are similar to those in Example 1-2.

[Example 1-2]

2 g of the collagen-SA-introduced-MA-PVA117(2.0) gel particles (swollen with ion-exchanged water) prepared in Synthesis Example 1-b were subjected to drying, water content measurement, alcohol solvent content measurement, and gamma ray irradiation in the same manner as in Example 1-1, affording a sterilized amide linkage-conjugated hydrogel. The measurement results of the water content and the content of the alcohol solvent are shown in Table 1. The degree of cohesion of the dry hydrogel-forming article obtained in Example 1-2 was 5.3%, and the breakage rate was 0%.

[Comparative Example 1-1]

2 g of the gelatin-SA-introduced-MA-PVA117(2.0) gel particles (swollen with ion-exchanged water) prepared in Synthesis Example 1-a were spread on a petri dish and naturally dried in air at room temperature for 2.7 hours. The water content of the dry particles obtained was measured with a heat-dry moisture analyzer (manufactured by A&D Co., Ltd.), and found to be 72 mass%. The dry gel particles were put in a 15 mL centrifuge tube, and further enclosed in an aluminum-metallized bag to prevent evaporation of water, and sterilized by irradiation with gamma rays (25 kGy) at KOGA ISOTOPE, Ltd., affording an amide linkage-conjugated hydrogel. The measurement results of water content, etc. are shown in Table 1.

[Comparative Examples 1-2 to 1-4]

Water content measurement and irradiation with gamma rays were performed in the same manner as in Comparative Example 1-1 except that the time for natural drying of 2 g of gelatin-SA-introduced-MA-PVA117(2.0) gel particles (swollen in ion-exchanged water) was changed to 3.7, 4.5, and 5.5 hours, affording sterilized amide linkage-conjugated hydrogels. The measurement results of water content are shown in Table 1.

**EP 4 671 292 A1**

[Comparative Example 1-5]

2 g of gelatin-SA-introduced-MA-PVA117(2.0) gel particles (swollen in ion-exchanged water) were spread on a petri dish and vacuum-dried at room temperature overnight. Water content measurement and irradiation with gamma rays were performed in the same manner as in Comparative Example 1-1, affording a sterilized amide linkage-conjugated hydrogel. The measurement results of water content are shown in Table 1. The degree of cohesion of the dry hydrogel-forming article obtained in Example 1-5 was 99.8%.

[Comparative Examples 1-6 to 1-7]

Water content measurement and irradiation with gamma rays were performed in the same manner as in Comparative Example 1-1 except that natural drying of 2 g of gelatin-SA-introduced-MA-PVA117(2.0) gel particles (swollen in ion-exchanged water) was not performed (Comparative Example 1-6) or the time for natural drying was changed 0.5 hours (Comparative Example 1-7), affording sterilized amide linkage-conjugated hydrogels. The measurement results of water content are shown in Table 1.

[Reference Example 1]

Drying, water content measurement, and irradiation with gamma rays were performed in the same manner as in Comparative Example 1-5 except that the gelatin-SA-introduced-Nor-PVA117(1.3) gel particles (swollen in ion-exchanged water) prepared in Synthesis Example 1-c were used, affording a sterilized amide linkage-conjugated hydrogel. The measurement results of water content are shown in Table 1. When the gel particles are subjected to drying, water content measurement, and irradiation with gamma rays in the same manner as in Example 1-1 to afford a sterilized amide linkage-conjugated hydrogel, it is likely that the gel hydrogel will exhibit an alcohol solvent content, a water content, a degree of cohesion, and a cell adhesion rate comparable to those in Example 1-1.

[Reference Example 2]

Drying, water content measurement, and irradiation with gamma rays were performed in the same manner as in Comparative Example 1-5 except that the gelatin-MA-PVA117(2.0)-SA(3.4) gel sheet (swollen in ion-exchanged water) prepared in Synthesis Example 1-d was used, affording a sterilized amide linkage-conjugated hydrogel. The measurement results of water content are shown in Table 1. When the gel sheet is subjected to drying, water content measurement, and irradiation with gamma rays in the same manner as in Example 1-1 to afford a sterilized amide linkage-conjugated hydrogel, it is likely that the gel hydrogel will exhibit an alcohol solvent content, a water content, a degree of cohesion, and a cell adhesion rate comparable to those in Example 1-1.

[Reference Example 3]

Drying, water content measurement, and irradiation with gamma rays were performed in the same manner as in Comparative Example 1-5 except that the gelatin-SA-introduced-GA crosslinked PVA117 gel sheet (swollen in ion-exchanged water) prepared in Synthesis Example 1-e were used, affording a sterilized amide linkage-conjugated hydrogel. The measurement results of water content are shown in Table 1. When the gel sheet is subjected to drying, water content measurement, and irradiation with gamma rays in the same manner as in Example 1-1 to afford a sterilized amide linkage-conjugated hydrogel, it is likely that the gel hydrogel will exhibit an alcohol solvent content, a water content, a degree of cohesion, and a cell adhesion rate comparable to those in Example 1-1.

[Example 1-3]

Drying, water content measurement, and irradiation with gamma rays were performed in the same manner as in Example 1-1 except that the gelatin-urethane-MA-PVA117(2.0) gel particles (swollen in ion-exchanged water) prepared in Synthesis Example 1-f were used, affording a sterilized conjugated hydrogel. The measurement results of water content, etc. are shown in Table 1. The degree of cohesion and the breakage rate of the dry hydrogel-forming article obtained in Example 1-3 are similar to those in Example 1-2.

[Method for evaluating conjugated hydrogels obtained in Examples and Comparative Examples]

25

<Evaluation of cell adhesiveness (particle)>

Poly(2-hydroxyethyl methacrylate) was dissolved in 95% ethanol at a concentration of 30 mg/mL, and 200 $\mu$L of this solution was dispensed into each well of a 24-well plate for cell culture (manufactured by IWAKI) and dried in a clean bench. Next, the sterilized amide linkage-conjugated hydrogel (spherical particles) obtained in Example 1-1 was immersed in phosphate buffered saline (PBS) for 1 hour, whereby the particles were swollen. 200 $\mu$L of the gel particles were placed in one well of the coated well plate, and 500 $\mu$L of DMEM medium supplemented with 10% fetal bovine serum was added to that well. $1.9 \times 10^5$ NIH/3T3 cells (purchased from ATCC) proliferated by pre-culture were added thereto and cultured at a carbon dioxide concentration of 5% under saturated water vapor pressure at 37°C. 24 hours after the culture, a photograph of the center of the culture well was taken with an inverted microscope equipped with a camera and image consolidation software, and the number of particles to which cells were adhered and extended was counted. As a result, adhesion and extension of cells were observed in 132 particles out of 200 gel particles. In this case, the cell adhesion rate (%) was defined by the percentage of the number of particles in which adhesion and extension of cells were observed out of 200 particles. The cell adhesion rate was 91% (Table 1).

The sterilized conjugated hydrogels (spherical particles) obtained in Examples 1-2 and 1-3, Comparative Examples 1-1 to 1-7, and Reference Example 1 were evaluated for cell adhesion in the same manner as in the Evaluation of cell adhesiveness (particle) described above. The results are shown in Table 1.

<Evaluation of cell adhesiveness (sheet)>

The sterilized amide linkage-conjugated hydrogel (sheet) obtained in Reference Example 2 was immersed in PBS for 1 hour, whereby the gel sheet was swollen, then the gel sheet was punched into a circle with a hole punch having a diameter of 15 mm, and the punched piece was placed on the bottom surface of a 24-well plate. Further, 500 $\mu$L of a DMEM medium containing 10% fetal bovine serum was added to the same well. In addition, on the same plate, a well containing 500 $\mu$L of only a medium without addition of the gel sheet was also prepared. Subsequently, $1.9 \times 10^5$ NIH/3T3 cells proliferated by pre-culture were added to the well containing the gel sheet and the well containing no gel sheet, and cultured at a carbon dioxide concentration of 5% under saturated water vapor pressure at 37°C. 24 hours after the culture, the medium in the wells was removed with an aspirator, and 500 $\mu$L of a fresh medium was added, followed by adding 50 $\mu$L of Cell Counting Kit-8, which is a reagent for measuring cell proliferation/cytotoxicity (CCK-8, manufactured by DOJINDO LABORATORIES). At the same time, 500 $\mu$L of a fresh medium and 50 $\mu$L of CCK-8 were added to unused wells that contained no gel sheet and no cells, and the well plate was incubated for 1 hour at a carbon dioxide concentration of 5% under saturated water vapor pressure at 37°C. After the incubation, 100 $\mu$L of the medium was taken from each well containing CCK-8 and transferred to a 96-well plate, and absorbance at 450 nm was measured with a microplate reader (ARVO, manufactured by PerkinElmer). With the absorbance determined based on the wells containing only cells taken as a cell adhesion rate of 100%, and the absorbance determined based on the wells containing only the medium taken as a cell adhesion rate of 0%, the cell adhesion rate was calculated from the absorbance determined based on the wells containing the gel sheet and cells and found to be 90% (Table 1).

The cell adhesiveness of the sterilized amide linkage-conjugated hydrogel obtained in Reference Example 3 was evaluated by the same method as in Reference Example 2, and the cell adhesion rate was found to be 87% (Table 1).

<Hydrogel swellability>

10 parts by mass of the dry hydrogel-forming articles obtained in Example 1-2 and Comparative Example 1-5 were each placed in 90 parts by mass of water and left to stand for 1.5 hours to be swollen, and then whether or not fragments of the hydrogel were generated, whether or not breakage occurred on the surface, and the like were visually observed. The observation results were evaluated according to the following criteria A to D. The result of Example 1-2 was A, and the result of Comparative Example 1-5 was C.

A: There is no generation of fragments of the hydrogel and no breakage of the hydrogel surface
B: Generation of fragments of the hydrogel and/or breakage of the hydrogel surface slightly occurred merely at a problem-free level
C: Generation of fragments of the hydrogel and/or breakage of the hydrogel surface occurred
D: Significant generation of fragments of the hydrogel and/or significant breakage of the hydrogel surface occurred

[Table 1]

| | No. | Vinyl alcohol polymer | Active group introduction rate [mol%] | Conjugation density of biologically active substance [$\mu$g/100 mg] | Content [wt%] | | Sterility | Cohesion | Cell adhesion rate |
|---|---|---|---|---|---|---|---|---|---|
| | | | | | Water | Alcohol solvent | | | |
| Example | 1-1 | MA-PVA117(2.0) | 10.9 | 48.5 | 0.1 or less | 0.05 | ○ | ○ | 91 |
| | 1-2 | MA-PVA117(2.0) | 10.9 | 48.5 | 0.1 or less | 0.05 | ○ | ○ | 90 |
| | 1-3 | MA-PVA117(2.0) | 13.5 | 8.3 | 0.1 or less | 0.05 | ○ | ○ | 85 |
| Comparative Example | 1-1 | MA-PVA117(2.0) | 10.9 | 48.5 | 72 | 0 | ○ | × | 66 |
| | 1-2 | MA-PVA117(2.0) | 10.9 | 48.5 | 53 | 0 | ○ | × | 80 |
| | 1-3 | MA-PVA117(2.0) | 10.9 | 48.5 | 34 | 0 | ○ | × | 90 |
| | 1-4 | MA-PVA117(2.0) | 10.9 | 48.5 | 15 | 0 | ○ | × | 89 |
| | 1-5 | MA-PVA117(2.0) | 10.9 | 48.5 | 7 | 0 | ○ | × | 90 |
| | 1-6 | MA-PVA117(2.0) | 10.9 | 48.5 | 96 | 0 | ○ | × | 47 |
| | 1-7 | MA-PVA117(2.0) | 10.9 | 48.5 | 92 | 0 | ○ | × | 52 |
| Reference Example | 1 | Nor-PVA117(1.3) | 8.7 | 38.8 | 7 | - | ○ | × | 92 |
| | 2 | MA-PVA117(2.0)-SA(3.4) | 3.4 | 28.3 | 8 | - | ○ | × | 90 |
| | 3 | PVA117 | 9.8 | 18.2 | 7 | - | ○ | × | 87 |

As is apparent from the results of Examples 1-1 to 1-3 and Comparative Examples 1-1 to 1-7, with the present invention, it was possible to produce a dry hydrogel-forming article which has been sufficiently sterilized and in which cohesion during drying is suppressed. In addition, it has also been confirmed that the hydrogels, which are swollen products of the dry hydrogel-forming articles, had high cell adhesion rates as compared with the hydrogels of Comparative Example 1-6, Comparative Example 1-7, and the like, in which sterilization was performed in a state where the content of water was high.

The breakage rate was 0% in Example 1-2. In Examples 1-1 and 1-3, Comparative Examples 1-1 to 1-7, and Examples 2-1 to 2-8 to be described later, the same vinyl alcohol polymer as in Example 1-2 was used, and the degree of polymerization was equal to or higher than that in Example 1-2, and therefore it is likely that the same breakage rate as that in Example 1-2 will be exhibited.

[Synthesis Examples]

<Synthesis of vinyl alcohol polymer having ethylenically unsaturated group>

[Synthesis Example 2-1]

40 g (monomer repeating unit: 908 mmol) of PVA117 (raw material PVA) was placed in a separable flask equipped with a 1-L Dimroth condenser. 350 mL of dimethylsulfoxide (DMSO) was added, and stirring with a mechanical stirrer was started. The temperature was raised to 80°C by a water bath, and then stirring was continued at 80°C for 4 hours. After visually confirming that the raw material PVA was dissolved, 1.2 g (10.9 mol) of vinyl methacrylate was added and stirred with heating at 80°C, and the mixture was further stirred at 80°C for 3 hours. After being allowed to cool, the reaction solution was poured into 2 L of methanol with stirring. The stirring was stopped and the mixture was left to stand for 1 hour. The solid obtained was collected, and then further immersed in 1 L of methanol for 1 hour and thereby washed. This washing operation was performed three times in total. The collected solid was vacuum-dried at room temperature overnight, affording methacryloylated PVA117. The introduction rate of an ethylenically unsaturated group (methacryloyl group) of the methacryloylated PVA117 was 1.2 mol% based on the repeating units of the raw material PVA (the methacryloylated PVA117 is hereinafter abbreviated as "MA-PVA117(1.2)").

[Synthesis Examples 2-2 and 2-3]

Vinyl alcohol polymers having ethylenically unsaturated groups were produced in the same manner as in Synthesis Example 2-1 except that the addition amount of vinyl methacrylate was changed as shown in Table 2.

[Synthesis Example 2-4]

A vinyl alcohol polymer having ethylenically unsaturated groups was produced in the same manner as in Synthesis Example 2-1 except that raw material PVA (AF-17), in which itaconic acid was copolymerized and carboxy groups were introduced, was used as shown in Table 2.

[Synthesis Example 2-5]

10 g (monomer repeating unit: 220 mmol) of MA-PVA117(2.0) prepared in Synthesis Example 2-2 was placed in a separable flask equipped with a 0.5-L Dimroth condenser. 90 mL of dimethylsulfoxide (DMSO) was added, and stirring with a mechanical stirrer was started. The temperature was raised to 80°C by a water bath, and then stirring was continued at 80°C for 4 hours. After visually confirming that the raw material PVA was dissolved, the water bath was set to 60°C. After confirming that the internal temperature reached 60°C, 1.2 g (12.1 mmol) of triethylamine and 1.1 g (11 mmol) of succinic anhydride were added, and the mixture was further stirred at 60°C for 5 hours. After being allowed to cool, the reaction solution was poured into 0.5 L of methanol with stirring. The stirring was stopped and the mixture was left to stand for 1 hour. The solid obtained was collected, and then immersed in 0.5 L of methanol for 1 hour and thereby washed. This washing operation was performed three times in total. The collected solid was vacuum-dried at room temperature overnight, affording methacryloylated PVA117 with succinic acid introduced thereinto. The introduction rate of succinic acid (SA) was 3.4 mol% based on the repeating units of MA-PVA117(2.0) (the methacryloylated PVA117 is hereinafter abbreviated as "MA-PVA117(2.0)-SA(3.4)").

[Synthesis Example 2-6]

60 g (monomer repeating unit: 1.36 mol) of PVA117 (raw material PVA) was placed in a separable flask equipped with a 1-L Dimroth condenser. 540 mL of ion-exchanged water was added, and stirring with a mechanical stirrer was started. The temperature was raised to 80°C by a water bath, and then stirring was continued at 80°C for 4 hours. After visually confirming that the raw material PVA was dissolved, the temperature was lowered to 40°C. While the solution was stirred at 40°C, 2.5 g (20.5 mmol) of 5-norbornene-2-carboxyaldehyde and 22 mL of a 10 vol% aqueous sulfuric acid solution were added, and the mixture was further stirred at 40°C for 4 hours. The mixture was allowed to cool, and then 80 mL of a 1 N aqueous NaOH solution was added for neutralization. The resulting mixture was placed in a dialysis membrane with a molecular weight cutoff of 3,500, and desalted (performed 4 times with 5 L of ion-exchanged water). The desalted aqueous solution was poured into 2 L of methanol with stirring, and the mixture was left to stand for 1 hour. The solid obtained was collected, and then further immersed in 1 L of methanol for 1 hour and thereby washed. The collected solid was vacuum-dried at room temperature overnight, affording norbornene-introduced PVA117. The introduction rate of ethylenically unsaturated groups (norbornenyl groups (Nor)) of the norbornene-introduced PVA117 was 1.3 mol% based on the repeating units of the raw material PVA (the norbornene-introduced PVA117 is hereinafter abbreviated as "Nor-PVA117(1.3)").

The vinyl alcohol polymers having ethylenically unsaturated groups produced in accordance with the methods described in Synthesis Examples 2-1 to 2-6 are summarized in Table 2.

[Table 2]

| | Vinyl alcohol polymer having ethylenically unsaturated groups | | | |
| --- | --- | --- | --- | --- |
| | Type | Raw material PVA | Ethylenically unsaturated group introduction rate [mol%] | Carboxy group [mol%] |
| Synthesis Example 2-1 | MA-PVA117(1.2) | PVA117 | 1.2 | - |
| Synthesis Example 2-2 | MA-PVA117(2.0) | PVA117 | 2 | - |
| Synthesis Example 2-3 | MA-PVA117(3.0) | PVA117 | 2.9 | - |
| Synthesis Example 2-4 | MA-AF-17(2.0) | AF-17 | 2.1 | 2.6 |
| Synthesis Example 2-5 | MA-PVA1 17(2.0)-SA(3.4) | PVA117 | 2 | 3.4 |
| Synthesis Example 2-6 | Nor-PVA117(1.3) | PVA117 | 1.3 | - |

<Production of molded article (particle, sheet)>

[Synthesis Example 2-A: Particle]

440 mL of ion-exchanged water was added to 60 g of MA-PVA117(1.2), and the mixture was dissolved at 80°C for 4 hours with stirring. After cooling to room temperature, potassium persulfate as a water-soluble thermal radical polymerization initiator was added to and dissolved in the aqueous solution of MA-PVA117(2.0) to attain a concentration of 0.1 mass%, whereby an uncrosslinked gel solution was prepared. A separable flask equipped with a 5-L Dimroth condenser was charged with 3300 mL of liquid paraffin and 8 g of Span80, and to this solution was slowly added the uncrosslinked gel solution. This mixed liquid was made into a W/O dispersion liquid while being stirred with a mechanical stirrer at 350 rpm, and the temperature was raised to 40°C by a water bath and then purged with nitrogen for 30 minutes. Thereafter, stirring was continued at 70°C for 3 hours. The mixture was cooled to room temperature, and the liquid paraffin in which hydrogel particles were dispersed was filtered through a mesh with an opening size of 100 $\mu$m. The hydrogel particles obtained were washed with 3 L in total of hexane to remove liquid paraffin, and the hydrogel particles obtained were classified into particle sizes of 180 to 300 $\mu$m using JIS standard sieves. Further, the hydrogel particles were charged into 1 L of acetone, dehydrated, and then dried at room temperature overnight under reduced pressure, affording dry hydrogel particles (hereinafter abbreviated as "MA-PVA117(1.2) gel particles").

[Synthesis Examples 2-B to 2-E: Particle]

Methacryloylated PVAs were produced in the same manner as in Synthesis Example 2-1 except that the vinyl alcohol polymers having an ethylenically unsaturated group synthesized in Synthesis Examples 2-2 to 2-5 were used.

[Synthesis Example 2-F: Particle]

In Synthesis Example 2-A, 440 mL of ion-exchanged water was added to 60 g of Nor-PVA117(1.3), and the mixture was dissolved at 80°C for 4 hours with stirring. After cooling to room temperature, 3.4 g of 3,6-dioxa-1,8-octanedithiol as a polythiol was added to the aqueous solution of Nor-PVA117(1.3), and the mixture was stirred. Potassium persulfate as a water-soluble thermal radical polymerization initiator was added to and dissolved in this solution to attain a concentration of 0.1 mass%, whereby an uncrosslinked gel solution was prepared.

Using this uncrosslinked gel solution, dry hydrogel particles were obtained in the same manner as in Synthesis Example 2-A (the dry hydrogel particles are hereinafter abbreviated as "Nor-PVA117(1.3) gel particles").

[Synthesis Example 2-G: Sheet]

44 mL of ion-exchanged water was added to 6 g of MA-PVA117(2.0)-SA(3.4), and the mixture was dissolved at 80°C for 4 hours with stirring. After cooling to room temperature, potassium persulfate as a water-soluble thermal radical polymerization initiator was added to and dissolved in the aqueous solution of MA-PVA117(2.0) to attain a concentration of 0.1 mass%, whereby an uncrosslinked gel solution was prepared. This uncrosslinked gel solution was poured between two glass plates with a 0.5 mm spacer interposed therebetween under nitrogen, and cured at 40°C for 3 hours. The cured hydrogel sheet (10 × 20 cm) having a thickness of 0.5 mm was washed five times with 300 mL of ion-exchanged water. Thereafter, the hydrogel sheet was charged into 1 L of acetone, dehydrated, and then dried at room temperature overnight under reduced pressure, affording a dry hydrogel sheet (hereinafter abbreviated as "MA-PVA117(2.0)-SA(3.4) gel sheet").

The molded articles produced in accordance with the methods described in Synthesis Examples 2-A to 2-G are summarized in Table 3.

[Table 3]

| | Molded article | |
|---|---|---|
| | Abbreviation | Vinyl alcohol polymer having ethylenically unsaturated groups |
| Synthesis Example 2-A | MA-PVA117(1.2) gel particle | MA-PVA117(1.2) |
| Synthesis Example 2-B | MA-PVA117(2.0) gel particle | MA-PVA117(2.0) |
| Synthesis Example 2-C | MA-PVA117(3.0) gel particle | MA-PVA117(3.0) |
| Synthesis Example 2-D | MA-AF-17(2.0) gel particle | MA-AF-17(2.0) |
| Synthesis Example 2-E | MA-PVA117(1.3) gel particle | MA-PVA117(1.3) |
| Synthesis Example 2-F | Nor-PVA117(1.3) gel particle | Nor-PVA117(1.3) |
| Synthesis Example 2-G | MA-PVA117(2.0)-SA(3.4) gel sheet | MA-PVA117(2.0)-SA(3.4) |

<Activation of hydroxy group>

[Synthesis Example 2-i: Introduction of succinic acid into molded article (particle)]

1.5 g of the dry MA-PVA117(1.2) gel particles (monomer repeating unit: 33 mmol) prepared in Synthesis Example 2-A was placed in a separable flask equipped with a 0.5-L Dimroth condenser. 100 mL of dimethylsulfoxide (DMSO) was added and stirring with a mechanical stirrer, whereby the gel particles were swollen. The mixture was stirred in a water bath at 60°C for 1 hour, then 364 mg (3.6 mmol) of triethylamine and 330 mg (3.3 mmol) of succinic anhydride were added, and the mixture was further stirred at 60°C for 5 hours. After being allowed to cool, the particles were collected by filtration and washed twice with 100 mL of DMSO and twice with 100 mL of water. Next, the particles were immersed three times in 100 mL of acetone to be dehydrated, and the particles obtained were dried under reduced pressure, affording dry hydrogel particles into which succinic acid had been introduced. The introduction rate of carboxy groups (succinic acid) (introduction density of carboxy groups) was 9.9 mol% based on the repeating units of MA-PVA117(1.2) (the resulting dry hydrogel particles are hereinafter abbreviated as "SA-introduced-MA-PVA117(1.2) gel particles").

[Synthesis Examples 2-ii to 2-iv: Introduction of succinic acid into molded article (particle)]

Dry hydrogel particles into which succinic acid had been introduced were obtained in the same manner as in Synthesis Example 2-i except that the gel particles prepared in Synthesis Examples 2-B to 2-C and 2-F were used as shown in Table 4. The results of the introduction rate of carboxy groups (succinic acid) (the introduction density of carboxy groups) are shown together in Table 4.

The introduction rates of carboxy groups (succinic acid) in the dry hydrogel particles produced in accordance with the methods described in Synthesis Examples 2-i to 2-iv are as shown in Table 4.

[Table 4]

| | Molded article (particle) with carboxy group introduced | |
| --- | --- | --- |
| | Particle abbreviation | Carboxy group (succinic acid) introduction rate [mol%] |
| Synthesis Example 2-i | SA-introduced-MA-PVA117(1.2) gel particle | 9.9 |
| Synthesis Example 2-ii | SA-introduced-MA-PVA117(2.0) gel particle | 10.3 |
| Synthesis Example 2-iii | SA-introduced-MA-PVA117(3.0) gel particle | 7.8 |
| Synthesis Example 2-iv | SA-introduced-MA-PVA117(1.3) gel particle | 9.5 |

<Conjugation>

[Example 2-1]

1 g of the dry SA-introduced-MA-PVA117(1.2) gel particles (succinic acid introduction amount: about 1.9 mmol) prepared in Synthesis Example 2-i were swollen in MES buffer (pH = 5.6) at room temperature overnight. The swollen gel particles were dispersed in 45 mL of MES buffer, 0.78 g (6.8 mmol) of N-hydroxysuccinimide and 0.65 g (3.4 mmol) of 1-(3-dimethylaminopropyl)-3-ethylcarbodiimide hydrochloride were added thereto with stirring using a magnetic stirrer, and the mixture was shaken at room temperature for 1 hour. The operation of washing the gel particles with 30 mL of MES buffer for 10 minutes was repeated three times, and the gel particles were added to 70 mL of a 1 mg/mL gelatin PBS solution. The operation of shaking at room temperature for 3 hours and washing the resulting hydrogel particles with 70 mL of ion-exchanged water (heated to 60°C) for 20 minutes was repeated twice. Further, immersion in 50 mL of ethanol was repeated three times, and then vacuum drying was performed at room temperature overnight, affording dry amide linkage gelatin-conjugated hydrogel particles (hereinafter abbreviated as "gelatin-SA-introduced-MA-PVA117(1.2) gel particles"). Some of the gelatin-SA-introduced-MA-PVA117(1.2) gel particles were immersed in excess PBS overnight and the amount of immobilized gelatin per gel weight (100 mg) (the conjugation density of the biologically active substance) was measured by the bicinchoninic acid (BCA) method (BCA Protein Assay Kit (manufactured by Takara Bio Inc.)) and found to be 48.2 μg/100 mg gel particles. Gamma-ray irradiation was performed in the same manner as in Comparative Example 1-1, affording a sterilized amide linkage-conjugated hydrogel. The water content and the content of the alcohol solvent of the resulting dry particles were calculated by $^1$H-NMR (deuterated DMSO solvent) in accordance with the measurement method described above. The measurement results of the water content, the content of the alcohol solvent, etc. are shown in Table 5.

[Examples 2-2 to 2-6]

Amide linkage gelatin-conjugated hydrogel particles were obtained in the same manner as in Example 2-1 except that the carboxy group-introduced hydrogel particles of Synthesis Examples 2-ii to 2-iv, 2-D, and 2-E were used. The results of measuring the amount of gelatin immobilized (the conjugation density of the biologically active substance) by the BCA method, and the results of the water content, the content of the alcohol solvent, etc. are shown in Table 5.

[Example 2-7]

Collagen-conjugated PVA particles were obtained in the same manner as in Synthesis Example 2-1 except that collagen was used instead of gelatin (the collagen-conjugated PVA particles are hereinafter abbreviated as "collagen-SA-introduced-MA-PVA117(1.2) gel particles"). The amount of gelatin immobilized (the conjugation density of the biologically active substance) was measured by the BCA method and found to be 53.2 μg/100 mg gel particles.

[Example 2-8]

<Gelatin-conjugated sheet>

An amide linkage gelatin-conjugated hydrogel sheet was obtained in the same manner as in Example 2-1 except that 1 g of the dry MA-PVA117(2.0)-SA(3.4) gel sheet prepared in Synthesis Example 2-G was used (the amide linkage gelatin-conjugated hydrogel sheet is hereinafter abbreviated as a "gelatinized-MA-PVA117(2.0)-SA (3.4) gel sheet"). The amount of gelatin immobilized (the conjugation density of the biologically active substance) was measured by the BCA method and found to be 20.0 µg/100 mg gel particles.

<Comparative Examples>

[Comparative Example 2-1]

When the breakage rate was measured in accordance with the above-described method using particles made of dextran commercially available as a carrier for gel filtration which did not include a crosslinked product of a vinyl alcohol polymer, the breakage rate was 40%.

[Comparative Example 2-2]

Particles made of the same commercially available dextran as that of Comparative Example 2-1 were irradiated with gamma rays at an irradiation dose of 25 kGy or more in the same manner as in Example 2-1, affording sterilized dextran particles. When the breakage rate of the resulting sterilized dextran particles was measured in accordance with the above-described method, the breakage rate was 60%.

[Comparative Example 2-3]

When the breakage rate was measured in accordance with the above-described method using particles made of cellulose commercially available as a carrier for gel filtration which did not include a crosslinked product of a vinyl alcohol polymer, the breakage rate was 10%.

[Comparative Example 2-4]

Particles made of the same commercially available cellulose as that of Comparative Example 2-3 were irradiated with gamma rays at an irradiation dose of 25 kGy or more in the same manner as in Example 2-1, affording sterilized dextran particles. When the breakage rate of the resulting sterilized dextran particles was measured in accordance with the above-described method, the breakage rate was 40%.

[Method for evaluating amide linkage-conjugated hydrogels obtained in Examples and Comparative Examples]

<Evaluation of cell adhesiveness (cell proliferation rate)>

30 mg of the dry SA-introduced-MA-PVA117(1.2) gel particles obtained in Example 2-1 were immersed in 5 mL of PBS overnight, the degree of swelling (weight in the swollen state/weight in the dry state) and the average particle size in the swollen state were measured, and the total surface area in the swollen state per 1 g of dry particles was calculated. The degree of swelling of the dry gel particles obtained in Example 2-1 was 8 or more. Subsequently, 30 mL of a DMEM medium supplemented with 10% fetal bovine serum was added to a 125-mL spinner flask (manufactured by Corning Incorporated), and the dry SA-introduced-MA-PVA117(1.2) gel particles prepared in Example 2-1 were weighed such that the total surface area would be 162 cm$^2$ based on the total surface area calculated beforehand, and were added thereto. Additionally, $8.1 \times 10^5$ NIH/3T3 cells (purchased from ATCC) proliferated by pre-culture were added and cultured in an incubator at a carbon dioxide concentration of 5% under saturated water vapor pressure at 37°C with stirring at a paddle rotation speed of 60 rpm. On the fourth day of the culture, all of the gel particles were collected and washed with PBS, and the cells were then detached from the gel particles by trypsin treatment. The cell density of the cell suspension obtained was counted with a hemocytometer, and the number of cells after the culture was calculated. When the number of cells after culture/initial number of cells was defined as a cell proliferation rate, the cell proliferation rate of the gel particles obtained in Example 2-1 was 10.6.

The results of measuring the cell proliferation rates of the gel particles of Examples 2-2 to 2-7 by the same method are shown in Table 5. The degrees of swelling of these dry gel particles and the dry gel particles of Examples 1-1 to 1-3 were all 8 to 11.

The cell proliferation rate of the gelatinized-MA-PVA117(2.0)-SA(3.4) gel sheet obtained in Example 2-8 was measured by the following method. The gel sheet was immersed in PBS overnight and swollen, and the gel sheet was then punched into a circle with a hole punch having a diameter of 34 mm, and the punched piece was placed on the bottom surface of a 6-well plate for cell culture made of polystyrene. Thereto was added 3 mL/well of a culture medium, and $4.9 \times 10^6$ NIH/3T3 cells proliferated by pre-culture were added and cultured in an incubator at a carbon dioxide concentration of 5% under saturated water vapor pressure at 37°C. On the fourth day of the culture, cells were detached and collected from the surface of the gel sheet by trypsin treatment. The number of cells after culture was counted, and the cell proliferation rate was calculated. As a result, the proliferation rate was 4.3.

<Sterilization validation ($VD_{max}$ method)>

[Example 3]

Gelatin-conjugated hydrogel particles were obtained in the same manner as in Synthesis Example 1-a except that operations were performed in a class 10,000 clean room, all the buffers and the ion-exchanged water used were those obtained through filtration and sterilization using a membrane filter with a pore size of 0.2 $\mu$m, and 30 g of the dry SA-introduced-MA-PVA117(2.0) gel particles prepared in Synthesis Example 1-i were used (the gelatin-conjugated hydrogel particles are hereinafter abbreviated as "gelatin-SA-introduced-MA-PVA117(2.0) gel particles (clean room)"). The amount of immobilized gelatin per gel weight (100 mg) was measured by the bicinchoninic acid (BCA) method, and a conjugation density of the biologically active substance was 56.3 $\mu$g/100 mg gel particles. The swollen gel particles obtained were dried in the same manner as in Example 1-1. The water content measured in the same manner as in Example 1-1 was 0.1 wt%.

The dry hydrogel particles obtained above were sterilized by $VD_{max}25$. Specifically, 1 g of the dry hydrogel particles were placed in a sterilized 25-mL centrifuge tube and covered with a lid. In this manner, 25 tubes of dry hydrogel particles kept in a container were prepared. The average bioburden of 10 tubes among these was measured and found to be 7.6. A sterility test was performed by irradiating 10 tubes with gamma rays with a dose of 6.9 kGy in VDmax25, which corresponds to an average bioburden of 8.0, and 1 out of 10 tubes was positive (substantiation of SAL = $10^{-1}$). Because this result revealed that the sterilization dose was 25 kGy, five tubes each containing the dry hydrogel particles kept in a container were irradiated with gamma rays of 25 kGy or more, thereby affording sterilized dry hydrogel particles that satisfied SAL = $10^{-6}$.

[Table 5]

| | No. | Carboxy group-containing PVA gel-forming body | Carboxy group introduction density [mol% (based on all structural units)] | Conjugation density of biologically active substance [$\mu$g/100 mg hydrous hydrogel] | Content [wt%] | | Sterility | Cohesion | Cell proliferation rate (number of cells after culture/initial number of cells) |
|---|---|---|---|---|---|---|---|---|---|
| | | | | | Water | Alcohol solvent | | | |
| Example | 2-1 | SA-introduced-MA-PVA117(1.2) gel particle | 9.9 | 48.2 | 0.1 or less | 0.05 | ○ | ○ | 10.6 |
| | 2-2 | SA-introduced-MA-PVA117(2.0) gel particle | 10.3 | 56.2 | 0.1 or less | 0.05 | ○ | ○ | 3.8 |
| | 2-3 | SA-introduced-MA-PVA117(3.0) gel particle | 7.8 | 30.7 | 0.1 or less | 0.05 | ○ | ○ | 5.2 |
| | 2-4 | SA-introduced-MA-PVA117(1.3) gel particle | 9.5 | 45.3 | 0.1 or less | 0.05 | ○ | ○ | 9.3 |
| | 2-5 | MA-AF-17 (2.0) gel particle | 2.6 | 8 | 0.1 or less | 0.05 | O | ○ | 4 |
| | 2-6 | MA-PVA117(2.0)-SA(3.4) gel particle | 3.4 | 40.5 | 0.1 or less | 0.05 | ○ | ○ | 5.8 |
| | 2-7 | SA-introduced-MA-PVA117(1.2) gel particle | 9.9 | 53.2 | 0.1 or less | 0.05 | ○ | ○ | 8.3 |
| | 2-8 | MA-PVA117(2.0)-SA(3.4) gel sheet | 3.4 | 20 | 0.1 or less | 0.05 | O | ○ | 4.3 |
| Comparative Example | 2-1 | Dextran particle | - | - | - | - | × | - | - |
| | 2-2 | Dextran particle | - | - | - | - | ○ | - | - |
| | 2-3 | Cellulose particle | - | - | - | - | × | - | - |
| | 2-4 | Cellulose particle | - | - | - | - | ○ | - | - |

As is apparent from the results of Examples 2-1 to 2-8, with the present invention, it was possible to produce a dry hydrogel-forming article which has been sufficiently sterilized and in which cohesion during drying is suppressed. In addition, it was also confirmed that the hydrogel which was a swollen product of the dry hydrogel had a high cell proliferation rate. On the other hand, in the case of the particles of Comparative Examples 2-1 to 2-4, in which no crosslinked product of a polyvinyl alcohol polymer was contained, it was confirmed that the strength of the hydrogel was not sufficient, and further, in the case of performing sterilization using gamma rays, the particles were further broken, and the strength was low.

**Claims**

1. A dry hydrogel-forming article comprising a crosslinked product of a vinyl alcohol polymer and an alcohol solvent, wherein

   a content of the alcohol solvent based on an amount of the dry hydrogel-forming article is 0.01 mass% or more and 15 mass% or less, and a content of water based on an amount of the dry hydrogel-forming article is 0 mass% or more and 70 mass% or less, and
   the dry hydrogel-forming article has a sterility assurance level SAL of $1 \times 10^{-6}$ or less and a degree of cohesion of 10% or less.

2. The dry hydrogel-forming article according to claim 1, wherein the following condition 1 is satisfied,
   Condition 1:
   when 100 parts by mass of water and 1 part by mass of the dry hydrogel-forming article are placed in a glass vial and stirred at a speed of 400 rpm for 3 hours using a stirrer chip having a length of 80% or more of a bottom diameter of the glass vial, a breakage rate of a swollen hydrogel-forming article is 5% or less.

3. The dry hydrogel-forming article according to claim 1 or 2, wherein a degree of polymerization of the vinyl alcohol polymer is 450 or more.

4. The dry hydrogel-forming article according to any one of claims 1 to 3, wherein the vinyl alcohol polymer has carboxy groups, and an introduction rate of carboxy groups is 0.1 to 50 mol% based on all structural units constituting the vinyl alcohol polymer.

5. The dry hydrogel-forming article according to any one of claims 1 to 4, wherein the vinyl alcohol polymer has ethylenically unsaturated groups, and an introduction rate of the ethylenically unsaturated groups is 0.01 to 10 mol% based on all structural units constituting the vinyl alcohol polymer.

6. The dry hydrogel-forming article according to claim 5, wherein the ethylenically unsaturated group is at least one selected from the group consisting of a vinyl group, a (meth)acryloyl group, a (meth)acryloylamino group, a vinylphenyl group, a norbornenyl group, and derivatives thereof.

7. The dry hydrogel-forming article according to any of claims 1 to 6, wherein the dry hydrogel-forming article is an indefinitely shaped particle, a spherical particle, a finely molded article, an article with any shape formed with a 3D printer, a sheet, a fiber, a hollow fiber, a porous monolith, or a coated article.

8. The dry hydrogel-forming article of any of claims 1 to 7, wherein the dry hydrogel-forming article is a spherical particle.

9. The dry hydrogel-forming article according to any one of claims 1 to 8, wherein a biologically active substance is conjugated.

10. The dry hydrogel-forming article according to any one of claims 1 to 9, wherein a biologically active substance is conjugated to a crosslinked product of a vinyl alcohol polymer by covalent bonding.

11. A hydrogel being a swollen product of the dry hydrogel-forming article according to any one of claims 1 to 10.

12. The hydrogel of claim 11, wherein the hydrogel is a spherical particle having a particle size of 10 to 5000 μm.

13. A method for producing the dry hydrogel-forming article according to any one of claims 1 to 10, comprising the steps of:

preparing a preliminary hydrogel including a crosslinked product of a vinyl alcohol polymer;
immersing the preliminary hydrogel in an alcohol solvent, and then drying the preliminary hydrogel to obtain a dry preliminary hydrogel; and
sterilizing the dry preliminary hydrogel until a sterility assurance level SAL is $1 \times 10^{-6}$ or less.

14. The method for producing the dry hydrogel-forming article according to claim 13, wherein radiation sterilization is performed in the step of sterilizing until the sterility assurance level SAL is $1 \times 10^{-6}$ or less.

15. The method for producing the dry hydrogel-forming article according to claim 14, wherein a radiation dose in the radiation sterilization is 8.2 kGy or more.

## INTERNATIONAL SEARCH REPORT

| International application No. |
|---|
| **PCT/JP2024/005065** |

**A. CLASSIFICATION OF SUBJECT MATTER**

*C08F 299/00*(2006.01)i; *A61K 9/06*(2006.01)i; *A61K 9/14*(2006.01)i; *A61K 38/39*(2006.01)i; *A61K 47/58*(2017.01)i; *A61K 47/69*(2017.01)i; *A61L 27/16*(2006.01)i; *A61P 43/00*(2006.01)i; *C08F 6/00*(2006.01)i; *C08J 7/00*(2006.01)i; *C08J 7/02*(2006.01)i; *C08K 5/05*(2006.01)i; *C08L 29/04*(2006.01)i; *C09D 129/04*(2006.01)i

FI: C08F299/00; C08K5/05; A61K9/06; A61K9/14; A61K47/69; A61K47/58; A61P43/00 111; A61K38/39; A61L27/16; C08L29/04 G; C08J7/02 A CEX; C08J7/00 302; C08F6/00; C09D129/04

According to International Patent Classification (IPC) or to both national classification and IPC

**B. FIELDS SEARCHED**

Minimum documentation searched (classification system followed by classification symbols)

C08F299/00; C08J3/02-3/11

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | US 2011/0054622 A1 (THE GENERAL HOSPITAL CORPORATION) 03 March 2011 (2011-03-03) | 1-15 |
| A | US 2015/0045909 A1 (MURATOGLU, Orhun K.) 12 February 2015 (2015-02-12) | 1-15 |
| A | JP 2010-538322 A (NOVARTIS AG) 09 December 2010 (2010-12-09) | 1-15 |
| A | JP 62-32110 A (CIBA GEIGY AG) 12 February 1987 (1987-02-12) | 1-15 |
| A | JP 2014-12851 A (BIOCOMPATIBLES UK LIMITED) 23 January 2014 (2014-01-23) | 1-15 |
| A | JP 2019-35043 A (KURARAY CO., LTD.) 07 March 2019 (2019-03-07) | 1-15 |
| P, A | CN 116854946 A (WUHAN TEXTILE UNIVERSITY) 10 October 2023 (2023-10-10) | 1-15 |

☐ Further documents are listed in the continuation of Box C.　　☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **12 April 2024** | **23 April 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

# EP 4 671 292 A1

**INTERNATIONAL SEARCH REPORT**
Information on patent family members

International application No.

**PCT/JP2024/005065**

| Patent document cited in search report | | | Publication date (day/month/year) | Patent family member(s) | | | Publication date (day/month/year) |
|---|---|---|---|---|---|---|---|
| US | 2011/0054622 | A1 | 03 March 2011 | WO | 2009/032921 | A1 | |
| US | 2015/0045909 | A1 | 12 February 2015 | WO | 2012/118662 | A2 | |
| JP | 2010-538322 | A | 09 December 2010 | JP | 2014-146050 | A | |
| | | | | US | 8647658 | B2 | |
| | | | | US | 2014/0096484 | A1 | |
| | | | | US | 2009/0059165 | A1 | |
| | | | | WO | 2009/032132 | A1 | |
| | | | | EP | 2990837 | A1 | |
| | | | | AR | 68123 | A | |
| | | | | KR | 10-2010-0055515 | A | |
| | | | | CN | 101790694 | A | |
| | | | | MX | 2010002342 | A | |
| | | | | TW | 200911306 | A | |
| | | | | RU | 2010112234 | A | |
| | | | | CA | 2692778 | A1 | |
| | | | | SG | 184705 | A | |
| | | | | AU | 2008295493 | A | |
| | | | | TW | 201402157 | A | |
| | | | | BR | PI0816104 | A | |
| | | | | CN | 104758974 | A | |
| | | | | AR | 99990 | A | |
| | | | | ZA | 200908849 | B | |
| | | | | SG | 10201500807P | A | |
| | | | | AU | 2013224663 | A | |
| JP | 62-32110 | A | 12 February 1987 | US | 4665123 | A | |
| | | | | EP | 216074 | A2 | |
| | | | | MX | 3292 | A | |
| | | | | PT | 83092 | A | |
| | | | | BR | 8603616 | A | |
| | | | | DK | 361686 | A | |
| | | | | HK | 192395 | A | |
| | | | | IE | 59807 | B | |
| | | | | IN | 168075 | A | |
| | | | | ZA | 8605688 | A | |
| | | | | ES | 2001047 | A | |
| | | | | AR | 244716 | A | |
| | | | | GR | 862018 | A | |
| | | | | AU | 6068386 | A | |
| | | | | IN | 168075 | B | |
| | | | | ZA | 865688 | B | |
| | | | | GR | 862018 | B | |
| JP | 2014-12851 | A | 23 January 2014 | JP | 2003-527402 | A | |
| | | | | JP | 2003-527172 | A | |
| | | | | JP | 2003-527173 | A | |
| | | | | US | 2001/0036451 | A1 | |
| | | | | US | 2001/0051670 | A1 | |
| | | | | US | 2001/0056301 | A1 | |
| | | | | US | 2003/0211073 | A1 | |
| | | | | US | 2003/0223956 | A1 | |
| | | | | US | 2005/0129656 | A1 | |

Form PCT/ISA/210 (patent family annex) (July 2022)

**INTERNATIONAL SEARCH REPORT**
Information on patent family members

International application No.

**PCT/JP2024/005065**

| Patent document cited in search report | | | Publication date (day/month/year) | Patent family member(s) | | | Publication date (day/month/year) |
|---|---|---|---|---|---|---|---|
| | | | | US | 2009/0209658 | A1 | |
| | | | | WO | 2001/068720 | A1 | |
| | | | | WO | 2001/068721 | A1 | |
| | | | | WO | 2001/068722 | A1 | |
| | | | | WO | 2002/072166 | A1 | |
| | | | | AU | 4360301 | A | |
| | | | | AU | 4361601 | A | |
| | | | | AU | 4566001 | A | |
| | | | | CA | 2402773 | A1 | |
| | | | | AU | 2001243603 | B | |
| | | | | AU | 2001243616 | B | |
| | | | | AU | 2001245660 | B | |
| JP | 2019-35043 | A | 07 March 2019 | (Family: none) | | | |
| CN | 116854946 | A | 10 October 2023 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002527206 A
- WO 2020105708 A
- WO 2020153382 A

**Non-patent literature cited in the description**

- *Food Chemistry*, 2001, vol. 74, 281-288
- *Biotechnology Techniques*, 1997, vol. 11, 67-70
- *Journal of Biomedical Materials Research*, 2001, vol. 57, 217-223
- *Journal of Polymer Engineering*, 2017, vol. 37, 647-660
- *Journal of Applied Biomaterials*, 1991, vol. 2, 261-267
- *Biomaterials*, 2002, vol. 23, 4325-4332
- *Biomaterials*, 2012, vol. 33, 3880-3886
- *Scientific Reports*, 2017, vol. 7, 45146